(19) Europäisches Patentamt
European Patent Office
Office européen des brevets

(11) Veröffentlichungsnummer: **0 224 791 B1**

(12) **EUROPÄISCHE PATENTSCHRIFT**

(45) Veröffentlichungstag der Patentschrift: **09.01.91 Patentblatt 91/02**

(51) Int. Cl.$^5$: **C08G 65/22,** C08L 23/00, C08L 25/00, C08L 33/00, C09D 7/12

(21) Anmeldenummer: **86115962.2**

(22) Anmeldetag: **18.11.86**

(54) **Oligomere Diazaoxaspirodecane, ihre Herstellung und ihre Verwendung als Lichtschutzmittel für Polymere.**

Die Akte enthält technische Angaben, die nach dem Eingang der Anmeldung eingereicht wurden und die nicht in dieser Patentschrift enthalten sind.

(30) Priorität: **26.11.85 DE 3541664**

(43) Veröffentlichungstag der Anmeldung: **10.06.87 Patentblatt 87/24**

(45) Bekanntmachung des Hinweises auf die Patenterteilung: **09.01.91 Patentblatt 91/02**

(84) Benannte Vertragsstaaten: **AT BE CH DE FR GB IT LI NL SE**

(56) Entgegenhaltungen:
**EP-A- 0 028 318**
**DE-A- 3 104 294**

(73) Patentinhaber: **HOECHST AKTIENGESELLSCHAFT**
**Postfach 80 03 20**
**D-6230 Frankfurt am Main 80 (DE)**

(72) Erfinder: **Ertl, Josef, Dr.**
**Eichenstrasse 14**
**D-8857 Wertingen (DE)**
Erfinder: **Korbanka, Helmut, Dr.**
**Birkenstrasse 24**
**D-8901 Adelried (DE)**

EP 0 224 791 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue, sterisch gehinderte Aminlichtstabilisatoren, ihre Herstellung und ihre Verwendung zum Stabilisieren von synthetischen Polymeren gegen die Einwirkung von Licht, Wärme und Sauerstoff,

Bekannt ist die Umsetzung von Polyalkyldiazaspirodecanen mit Epichlorhydrin zu den entsprechenden Epoxiverbindungen, die sich durch Erhitzen in Polyether mit Polymerisationsgraden von bis zu ca. 50 überführen lassen (vgl. EP 28 318 = DE 29 41 004). Die polymeren Verbindungen weisen eine sehr geringe Flüchtigkeit auf und sind als Lichtstabilisatoren für organische Polymere geeignet.

Weiterhin sind Umsetzungen von Diazaoxaspirodecanen mit Epoxiden schon beschrieben worden (vgl. DE 31 04 294). Dabei entstehen monomere verbindungen, die durch Umsetzung mit bifunktionellen Verbindungen zu Oligomeren oder Polymeren umgewandelt, als Lichtstabilisatoren für synthetische Polymere verwendbar sind.

Die vorliegende Erfindung betrifft nun Verbindungen der Formel (I)

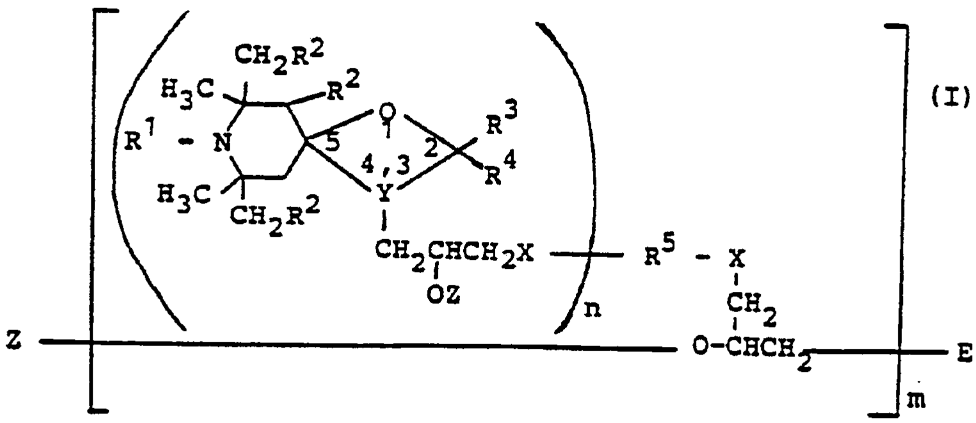

(I)

worin

n eine ganze Zahl von 1 bis 4, vorzugsweise 1, ist,

m eine ganze Zahl von 2 bis 100 vorzugsweise 2 bis 20 ist,

$R^1$ Wasserstoff, $C_1$-bis $C_4$-Alkyl, Benzyl, Allyl, $C_2$-bis $C_{30}$-Alkanoyl, $C_2$-bis $C_{20}$-Alkenoyl, $C_7$-bis $C_{11}$-Aroyl, $C_8$-bis $C_{14}$-Arylalkanoyl oder $C_8$-bis $C_{20}$-Alkylaryl, vorzugsweise Wasserstoff, $C_1$-bis $C_4$-Alkyl, $C_2$-bis $C_{30}$ Alkanoyl, insbesondere Wasserstoff oder Acyl, ganz besonders bevorzugt Wasserstoff ist,

$R^2$ Wasserstoff oder $C_1$-bis $C_4$-Alkyl, vorzugweise Wasserstoff ist,

$R^3$ und $R^4$ gleich oder verschieden sind und Wasserstoff, $C_1$-bis $C_{13}$-Alkyl, vorzugsweise $C_1$-bis $C_9$-Alkyl, unsubstituiertes oder durch Chlor oder $C_1$-bis $C_4$-Alkyl substituiertes Phenyl, unsubstitutiertes oder durch $C_1$ bis $C_4$-Alkyl substituiertes $C_7$-bis $C_{14}$-Aralkyl vorzugsweise $C_7$-bis $C_2$-Phenylalkyl oder zusammen mit dem sie bindenden Kohlenstoffatom einen unsubstituierten oder durch bis zu vier $C_1$-bis $C_4$-Alkylgruppen substituierten $C_5$-bis $C_{12}$-Cycloalkyl-oder Piperidinring bedeuten,

$R^5$ bei n = 1 $C_2$-bis $C_{18}$-Alkylen, vorzugsweise $C_2$-bis $C_{12}$-Alkylen, unsubstituiertes oder durch bis zu zwei $C_1$-bis $C_4$-, vorzugsweise $C_1$-Alkylgruppen substituiertes Phenylen, α, ω-Dicarboxi-$C_1$-bis $C_8$-Alkylen, einen Dicarboxy-$C_6$-Ring, $C_7$-bis $C_{14}$-Aralkylen, Cycloalkylen, Dicycloalkylen, Tricycloalkylen, Bismethylenmonocycloalkylen, Bismethylendicycloalkylen, Bismethylentricycloalkylen, Arylen, Bisarylenalkyl, wobei diese Reste auch durch Brom oder Chlor oder $C_1$-bis $C_4$-Alkylreste substituiert sein können, einen Rest

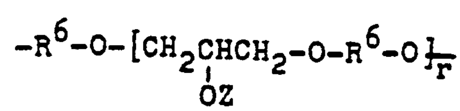

mit

r = 1 bis 20 und $R^6$ gleich $C_2$-bis $C_{12}$-bis $C_{12}$-Alkylen, Cycloalkylen, Dicycloalkylen, Tricycloalkylen, Bismethylenmonocycloalkylen, Bismethylendicycloalkylen, Bismethylentricycloalkylen, Arylen, Bisarylenalkyl, wobei diese Reste auch durch Brom oder Chlor bzw $C_1$-bis $C_4$-Alkylreste substituiert sein können, bedeutet

$R^5$ bei n = 2 den Rest eines trifunktionellen Alkohols oder Amins, den trifunktionellen Rest eines aliphatischen Alkohols, der noch weitere Hydroxylgruppen enthält, oder den trifunktionellen Rest eines Novolaks auf Basis Phenol, Kresol, Bisphenol-F oder Bisphenol A bedeutet,
$R^5$ bei n = 3 den tetrafunktionellen Rest eines aliphatischen Alkohols oder Amins, vorzugsweise den Pentaerythritylrest, oder eines Novolaks auf Basis Phenol, Kresol, Bisphenol-F oder Bisphenol-A oder den Rest

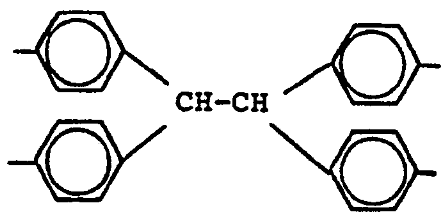

bedeutet,
$R^5$ bei n = 4 einen pentafunktionellen Rest eines Polyols, eines Polyamins oder eines Novolaks auf Basis Phenol, Kresol, Bisphenol-F oder Bisphenol-A bedeutet,

Y 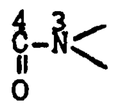 oder 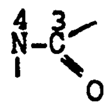 bedeutet und

die Ringpositionen 3, 4 in Formel I einnimmt,

X -O- oder 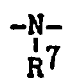 darstellt, wobei $R^7$

Wasserstoff, $C_1$- bis $C_{30}$-Alkyl, ein durch 1 bis 4 $C_1$- bis $C_4$-Alkylgruppen substituierter Piperidinring oder ein Rest der Formel (II)

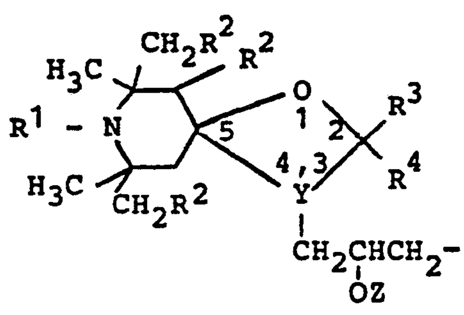 (II)

worin $R^1$, $R^2$, $R^3$, $R^4$, Y die oben angegebenen Bedeutungen haben, ist,

Z Wasserstoff, ein Rest der Formel (III)

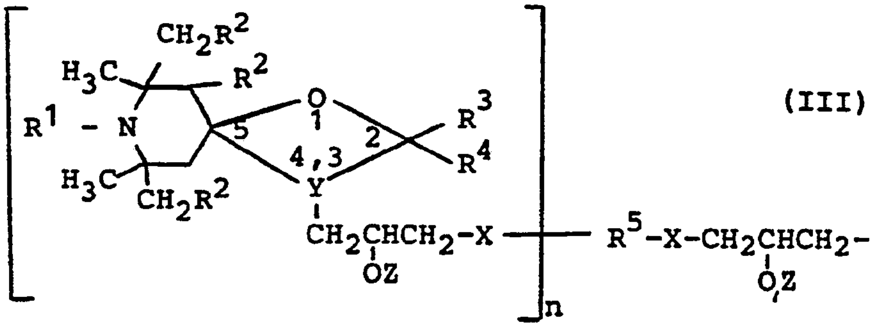

(III)

worin $R^1$, $R^2$, $R^3$, $R^4$, $R^5$, X, Y, Z und n die oben angegebenen Bedeutungen haben, oder ein Rest der Formel (II) ist,

E für Wasserstoff, Hydroxyl, einen Rest der Formel (IV), (V) oder (VI)

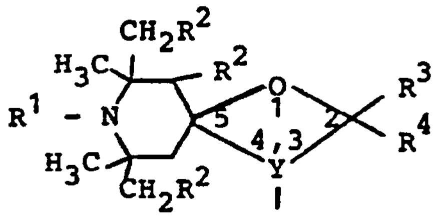

(IV)

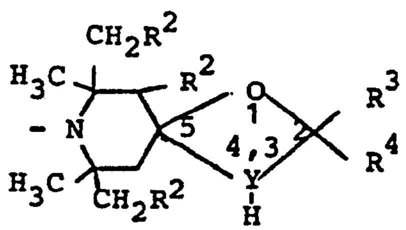

(V)

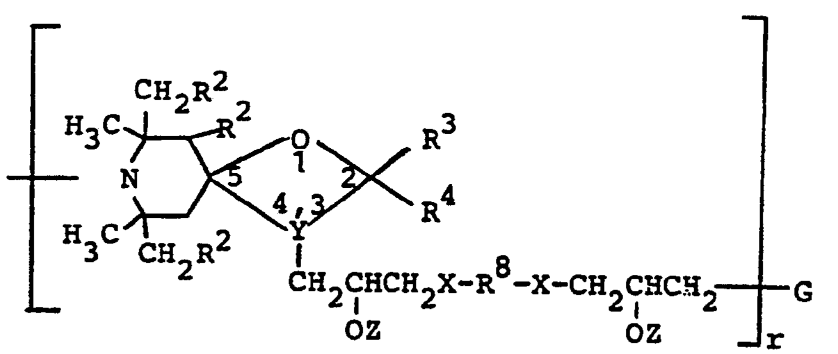

(VI)

worin $R^1$, $R^2$, $R^3$, $R^4$, $R^5$, X, Y und Z die oben angegebenen Bedeutungen haben, r eine ganze Zahl von 1 bis 100, vorzugsweise 1 bis 20, darstellt und G ein Rest der Formel (IV) oder (V) ist und $R^8$ die Bedeutung der Formel (VII)

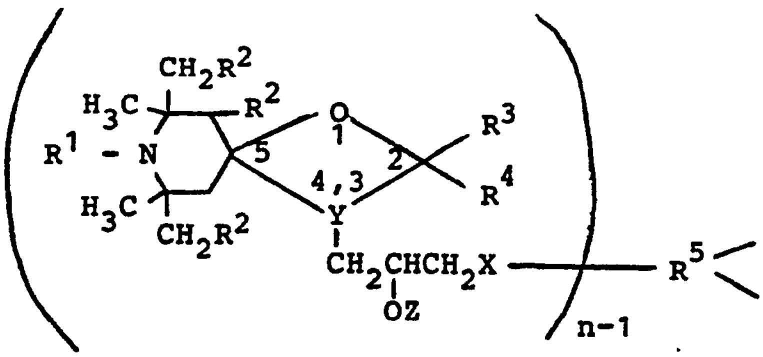

(VII)

worin $R^1$, $R^2$, $R^3$, $R^4$, $R^5$, X, Y, Z und n die oben angegebenen Bedeutungen haben, hat.

Diese neuen Lichtstabilisatoren der Formel I werden aus Diazaoxaspirodecanen der Formel (VIIIa) oder (VIIIb)

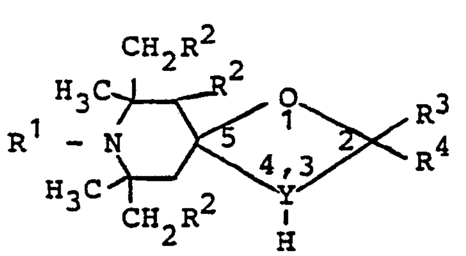

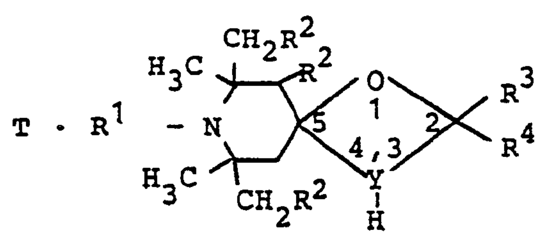

(VIIIa) (VIIIb)

worin $R^1$, $R^2$, $R^3$, $R^4$ und Y die oben angegebenen Bedeutungen haben und T eine nicht oxidierende Mineralsäure oder eine aliphatische oder aromatische Sulfon- oder Phosphonsäure, eine aliphatische Mono-, Di- oder Polycarbonsäure oder eine aromatische Mono- oder Dicarbonsäure ist, und Epoxiden der Formel (IXa) oder (IXb)

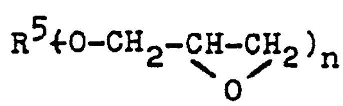

(IXa)

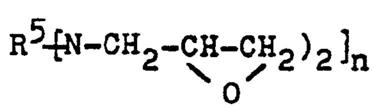

(IXb)

worin $R^5$ und n die oben angegebenen Bedeutungen haben, hergestellt, wobei die Verbindungen der Formel (VIII) mit einem Unterschuß von einem bis zu einem fünftel Äquivalent, bezogen auf die Epoxidgruppen in den Formeln (IXa) oder (IXb), eingesetzt werden. Diese Umsetzung erfolgt in einem inerten organischen Lösungsmittel, beispielsweise Toluol oder Xylol, in Gegenwart der 0,1- bis 1,5fach äquivalenten Menge einer Base bezogen auf die Verbindung (VIIIa) oder der 1,2- bis 2,5fach äquivalenten Menge einer Base bei Einsatz der Verbindungen (VIIIb), vorzugsweise mit der 0,3- bis 1,2fach bzw. 1,3- bis 2,2fach äquivalenten Menge an Natriumhydroxid oder Kaliumhydroxid, wobei gegebenenfalls ein Phasentransferkatalysator zugesetzt wird. Die Reaktionstemperatur liegt bei 40 bis 180°C, bevorzugt bei 50 bis 150°C, besonders

bevorzugt bei 80 bis 110°C.

Die nach diesem Reaktionsschritt erhaltenen Zwischenprodukte enthalten noch Epoxidgruppen, so daß diese Zwischenprodukte in Substanz oder in Lösung, gegebenenfalls in Gegenwart von Katalysatoren, in höhermolekulare Verbindungen überführt werden können. Dies kann z.B. durch Erhitzen dieser Zwischenprodukte auf 100 bis 240°C, vorzugsweise 140 bis 210°C, insbesondere 160 bis 200°C, erfolgen. Hierbei können auch Salze als Katalysatoren zugesetzt werden, wobei basische Verbindungen wie beispielsweise $BaCO_3$ die Polyetherbildung, saure Verbindungen, wie beispielsweise $NH_4Cl$, die Addition der Aminfunktion an die Epoxidgruppe fördern.

Zur Herstellung der Polymeren bzw. Oligomeren kann man auch so verfahren, daß man die epoxidgruppenhaltigen Zwischenprodukte nicht erst isoliert, sondern in der Reaktionsmischung auf die genannten höheren Temperaturen bringt und nach erfolgter Polymerisation bzw. Polyaddition aufarbeitet.

Die Polymerisations-/Polyadditionsgrade können sowohl durch das Verhältnis der Ausgangmaterialien zueinander als auch durch die Reaktionsbedingungen beeinflußt werden, so führt eine Erhöhung des Gehaltes an Epoxidverbindungen IX im Verhältnis zu den Diazaspirodecanen VIII zu höheren mittleren Molmassen. Ebenso führt eine Verlängerung der Polymerisations-/Polyadditionszeit sowie eine Erhöhung der Polymerisations-/Polyadditionstemperatur zu höhermolekularen Produkten.

Bei der Bildung höhermolekularer Produkte können hierbei prinzipiell folgende Reaktionen ablaufen :

– Polyetherbildung aus den Epoxidgruppen (Polymerisation),
– Addition der Aminfunktion an die Epoxidgruppen (Polyaddition) und/oder
– Addition vorhandener Hydroxylgruppen an die Epoxidgruppen (Polyaddition).

Überraschenderweise läßt die oben beschriebene Reaktionsführung die Herstellung von Verbindungen mit einer bestimmten mittleren Molmasse zu, wobei diese in weiten Grenzen gezielt variiert werden kann.

Gegenüber den Verbindung aus der DE-OS 31 04 294 besteht der große Vorteil darin, daß bei den erfindungsgemäßen Verbindungen das Arbeiten mit Epichlorhydrin, einem Stoff, der sich im Tierversuch eindeutig als krebserzeugend erwiesen hat, entfällt.

Die Ausgangsmaterialien der Formel (VIIIa) oder (VIIIb) sind an sich bereits gute Stabilisatoren, befriedigen jedoch besonders bezüglich der Verträglichkeit mit dem zu stabilisierenden Polymeren und der Flüchtigkeit nicht. Die erfindungsgemäßen Stabilisatoren weisen diese Nachteile nicht auf und zeigen überraschender Weise auch eine deutlich bessere antioxidative und lichtstabilisierende Wirkung.

Geeignete Verbindungen der Formel VIIIa sind beispielsweise :

1. 2-Butyl-7,7,9,9-tetramethyl-1-oxa-3,8-diaza-4-oxo-spiro-[4,5]-decan
2. 2-iso-Butyl-7,7,9,9-tetramethyl-1-oxa-3,8-diaza-4-oxo-spiro-[4,5]-decan
3. 2-Pentyl-7,7,9,9-tetramethyl-1-oxa-3,8-diaza-4-oxo-spiro-[4,5]-decan
4. 2-iso-Pentyl-7,7,9,9-tetramethyl-1-oxa-3,8-diaza-4-oxo-spiro-[4,5]-decan
5. 2-Hexyl-7,7,9,9-tetramethyl-1-oxa-3,8-diaza-4-oxo-spiro-[4,5]-decan
6. 2-Heptyl-7,7,9,9-tetramethyl-1-oxa-3,8-diaza-4-oxo-spiro-[4,5]-decan
7. 2-iso-Heptyl-7,7,9,9-tetramethyl-1-oxa-3,8-diaza-4-oxo-spiro-[4,5]-decan
8. 2-Nonyl-7,7,9,9-tetramethyl-1-oxa-3,8-diaza-4-oxo-spiro-[4,5]-decan
9. 2-iso-Nonyl-7,7,9,9-tetramethyl-1-oxa-3,8-diaza-4-oxo-spiro-[4,5]-decan
10. 2-Undecyl-7,7,9,9-tetramethyl-1-oxa-3,8-diaza-4-oxo-spiro-[4,5]-decan
11. 2-Phenyl-7,7,9,9-tetramethyl-1-oxa-3,8-diaza-4-oxo-spiro-[4,5]-decan
12. 2-(4-Chlor-phenyl)-7,7,9,9-tetramethyl-1-oxa-3,8-diaza-4-oxo-spiro-[4,5]-decan
13. 2-Ethyl-2,7,7,9,9-pentamethyl-1-oxa-3,8-diaza-4-oxo-spiro-[4,5]-decan
14. 2-Propyl-2,7,7,9,9-pentamethyl-1-oxa-3,8-diaza-4-oxo-spiro-[4,5]-decan
15. 2-iso-Propyl-2,7,7,9,9-pentamethyl-1-oxa-3,8-diaza-4-oxo-spiro-[4,5]-decan
16. 2-Butyl-2,7,7,9,9-pentamethyl-1-oxa-3,8-diaza-4-oxo-spiro-[4,5]-decan
17. 2-iso-Butyl-2,7,7,9,9-pentamethyl-1-oxa-3,8-diaza-4-oxo-spiro-[4,5]-decan
18. 2-Pentyl-2,7,7,9,9-pentamethyl-1-oxa-3,8-diaza-4-oxo-spiro-[4,5]-decan
19. 2-Hexyl-2,7,7,9,9-pentamethyl-1-oxa-3,8-diaza-4-oxo-spiro-[4,5]-decan
20. 2-Nonyl-2,7,7,9,9-pentamethyl-1-oxa-3,8-diaza-4-oxo-spiro-[4,5]-decan
21. 2,2,7,7,9,9-Hexamethyl-1-oxa-3,8-diaza-4-oxo-spiro-[4,5]-decan
22. 2,2,7,7,9,9-Heptamethyl-1-oxa-3,8-diaza-4-oxo-spiro-[4,5]-decan
23. 2,2-Diethyl-7,7,9,9-tetramethyl-1-oxa-3,8-diaza-4-oxo-spiro-[4,5]-decan
24. 2,2-Dipropyl-7,7,9,9-tetramethyl-1-oxa-3,8-diaza-4-oxo-spiro-[4,5]-decan
25. 2,2-Dibutyl-7,7,9,9-tetramethyl-1-oxa-3,8-diaza-4-oxo-spiro-[4,5]-decan
26. 2-Ethyl-2-pentyl-7,7,9,9-tetramethyl-1-oxa-3,8-diaza-4-oxo-spiro-[4,5]-decan
27. 2,2-Dibenzyl-7,7,9,9-tetramethyl-1-oxa-3,8-diaza-4-oxo-spiro-[4,5]-decan
28. 2,2,4,4-Tetramethyl-7-oxa-3,13-diaza-14-oxo-dispiro-[5,1,4,2]-tetradecan

29. 2,2,4,4-Tetramethyl-7-oxa-3,14-diaza-15-oxo-dispiro-[5,1,5,2]-pentadecan
30. 2,2,4,4-Tetramethyl-7-oxa-3,20-diaza-21-oxo-dispiro-[5,1,11,2]-heneicosan
31. 2,2,7,7,9,9-Hexamethyl-1-oxa-3,8-diaza-4-oxo-8-acetylspiro[-4,5]-decan
32. 2,2,4,4-Tetramethyl-7-oxa-3,14-diaza-15-oxo-3-acetyl-dispiro-[5,1,5,2]-pentadecan
33. 2,2,4,4-Tetramethyl-7-oxa-3,20-diaza-21-oxo-3-acetyl-dispiro-[5,1,11,2]-heneicosan
34. 2,7,7,9,9-Pentamethyl-1-oxa-4,8-diaza-3-oxo-spiro-[4,5]-decan
35. 2-Ethyl-7,7,9,9-tetramethyl-1-oxa-4,8-diaza-3-oxo-spiro-[4,5]-decan
36. 2-Propyl-7,7,9,9-tetramethyl-1-oxa-4,8-diaza-3-oxo-spiro[4,5]-decan
37. 2-Butyl-7,7,9,9-tetramethyl-1-oxa-4,8-diaza-3-oxo-spiro[4,5]-decan
38. 2-iso-Butyl-7,7,9,9-tetramethyl-1-oxa-4,8-diaza-3-oxo-spiro[4,5]-decan
39. 2-Pentyl-7,7,9,9-tetramethyl-1-oxa-4,8-diaza-3-oxo-spiro[4,5]-decan
40. 2-iso-Pentyl-7,7,9,9-tetramethyl-1-oxa-4,8-diaza-3-oxo-spiro-[4,5]-decan
41. 2-iso-Heptyl-7,7,9,9-tetramethyl-1-oxa-4,8-diaza-3-oxo-spiro-[4,5]-decan
42. 2-Phenyl-7,7,9,9-tetramethyl-1-oxa-4,8-diaza-3-oxo-spiro-[4,5]-decan
43. 2,2,7,7,9,9-Hexamethyl-1-oxa-4,8-diaza-3-oxo-spiro-[4,5]-decan
44. 2,2,7,7,8,9,9-Heptamethyl-1-oxa-4,8-diaza-3-oxo-spiro-[4,5]-decan
45. 2,2-Diethyl-7,7,9,9-tetramethyl-1-oxa-4,8-diaza-3-oxo-spiro[4,5]-decan
46. 2,2-Diethyl-7,7,8,9,9-pentamethyl-1-oxa-4,8-diaza-3-oxo-spiro-[4,5]-decan
47. 2,2-Dipropyl-7,7,9,9-tetramethyl-1-oxa-4,8-diaza-3-oxo-spiro-[4,5]-decan
48. 2,2-Dibutyl-7,7,9,9-tetramethyl-1-oxa-4,8-diaza-3-oxo-spiro-[4,5]-decan
49. 2,2-Dipentyl-7,7,9,9-tetramethyl-1-oxa-4,8-diaza-3-oxo-spiro-[4,5]-decan
50. 2-Ethyl-2,7,7,9,9-pentamethyl-1-oxa-4,8-diaza-3-oxo-spiro-[4,5]-decan
51. 2-Propyl-2,7,7,9,9-pentamethyl-1-oxa-4,8-diaza-3-oxo-spiro-[4,5]-decan
52. 2-iso-Propyl-2,7,7,9,9-pentamethyl-1-oxa-4,8-diaza-3-oxo-spiro-[4,5]-decan
53. 2-Butyl-2,7,7,9,9-pentamethyl-1-oxa-4,8-diaza-3-oxo-spiro-[4,5]-decan
54. 2-iso-Butyl-2,7,7,9,9-pentamethyl-1-oxa-4,8-diaza-3-oxo-spiro-[4,5]-decan
55. 2-Pentyl-2,7,7,9,9-pentamethyl-1-oxa-4,8-diaza-3-oxo-spiro-[4,5]-decan
56. 2-iso-Pentyl-2,7,7,9,9-pentamethyl-1-oxa-4,8-diaza-3-oxo-spiro-[4,5]-decan
57. 2-Hexyl-2,7,7,9,9-pentamethyl-1-oxa-4,8-diaza-3-oxo-spiro-[4,5]-decan
58. 2-Heptyl-2,7,7,9,9-pentamethyl-1-oxa-4,8-diaza-3-oxo-spiro-[4,5]-decan
59. 2-Nonyl-2,7,7,9,9-pentamethyl-1-oxa-4,8-diaza-3-oxo-spiro-[4,5]-decan
60. 2-Undecyl-2,7,7,9,9-pentamethyl-1-oxa-4,8-diaza-3-oxo-spiro-[4,5]-decan
61. 2-Ethyl-2-butyl-7,7,9,9-tetramethyl-1-oxa-4,8-diaza-3-oxo-spiro-[4,5]-decan
62. 2-Ethyl-2-pentyl-7,7,9,9-tetramethyl-1-oxa-4,8-diaza-3-oxo-spiro-[4,5]-decan
63. 2-Ethyl-2-iso-pentyl-7,7,9,9-tetramethyl-1-oxa-4,8-diaza-spiro-[4,5]-decan
64. 2,2,7,7,9,9-Hexamethyl-1-oxa-4,8-diaza-3-oxo-8-acetyl-spiro-[4,5]-decan
65. 2,2-Diethyl-7,7,9,9-tetramethyl-1-oxa-4,8-diaza-3-oxo-8-acetyl-spiro-[4,5]-decan
66. 2,2,4,4-Tetramethyl-7-oxa-3,14-diaza-13-oxo-dispiro-[5,1,4,2]-tetradecan
67. 2,2,4,4-Tetramethyl-7-oxa-3,15-diaza-14-oxo-dispiro-[5,1,5,2]-pentadecan
68. 2,2,4,4-Tetramethyl-7-oxa-3,21-diaza-20-oxo-dispiro-[5,1,11,2]-heneicosan

Geeignete Verbindung der Formel VIIIb sind die Salze aus den Verbindungen der Formel VIIIa mit Protonsäuren, z.B. Chlorwasserstoff, Schwefelsäure, Phosphorsäure usw., beispielsweise die Hydrochloride der oben angegebenen Verbindungen Nr. 1- 68. Zur Erläuterung seien folgende Beispiele genannt :

69. 2,2,7,7,9,9-Hexamethyl-1-oxa-3,8-diaza-4-oxo-spiro-[4,5]-decan-hydrochlorid
70. 2,2,4,4-Tetramethyl-7-oxa-3,14-diaza-15-oxo-dispiro-[5.1.5.2]-pentadecan-hydrochlorid
71. 2,2,4,4-Tetramethyl-7-oxa-3,20-diaza-21-oxo-dispiro [5.1.11.2]-heneicosan-hydrochlorid
72. 2,2,7,7,9,9-Hexamethyl-1-oxa-4,8-diaza-3-oxo-spiro-[4,5]-decan-hydrochlorid
73. 2,2-Diethyl-7,7,9,9-tetramethyl-1-oxa-4,8-diaza-3-oxo-spiro-[4,5]-decan-hydrochlorid
74. 2,2,4,4-Tetramethyl-7-oxa-3,14-diaza-13-oxo-dispiro-[5.1.4.2]-tetradecan-hydrochlorid
75. 2,2,4,4-Tetramethyl-7-oxa-3,15-diaza-14-oxo-dispiro-[5.1.5.2]-pentadecan-hydrochlorid
76. 2,2,4,4-Tetramethyl-7-oxa-3,21-diaza-20-oxo-dispiro-[5.1.11.2]-heneicosan-hydrochlorid

Beispiele für die Epoxide der Formel IX sind :

77. 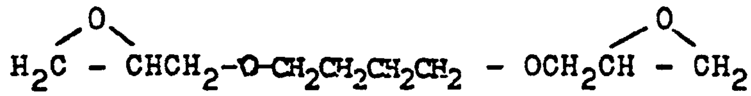

78. 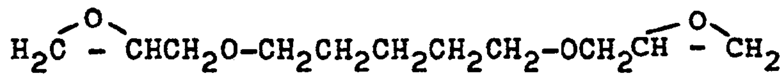

79. 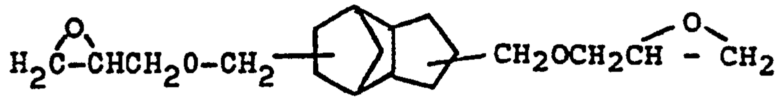

80. 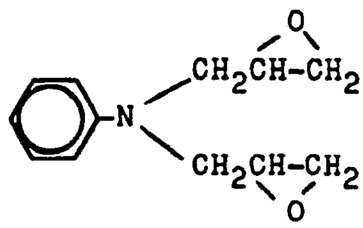

81. 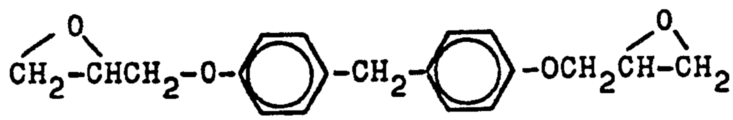

82. 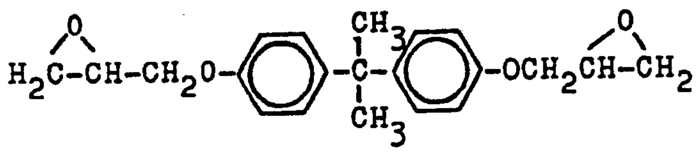

83. 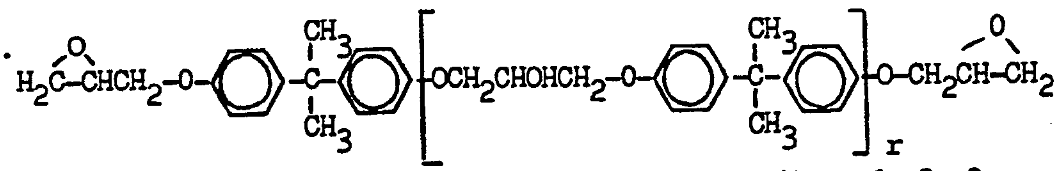

mit r= 1, 2, 3 ...

84. 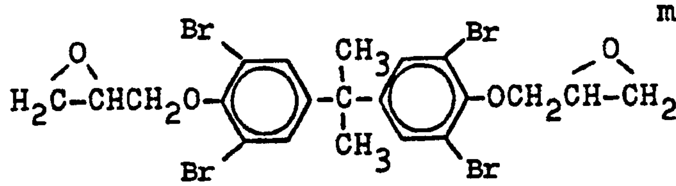

85. 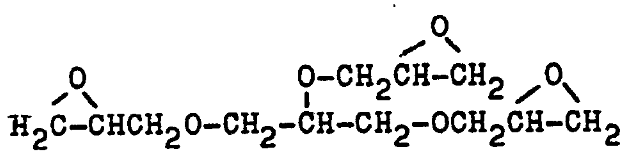

86. 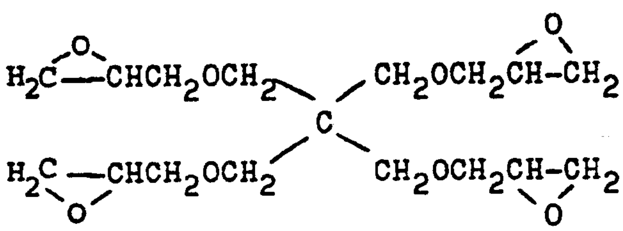

87. 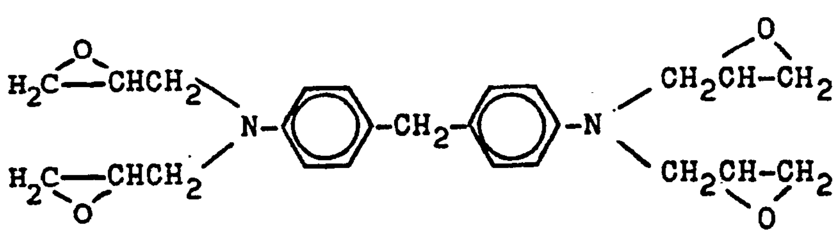

88. 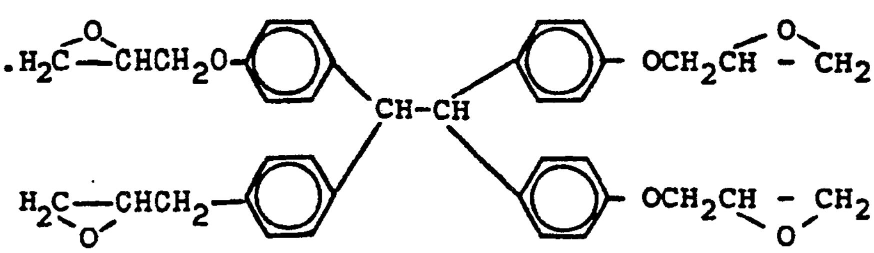

89. 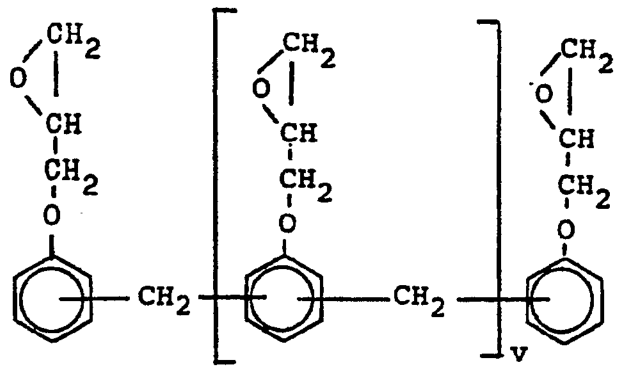

mit v= 1, 2, 3...

90. 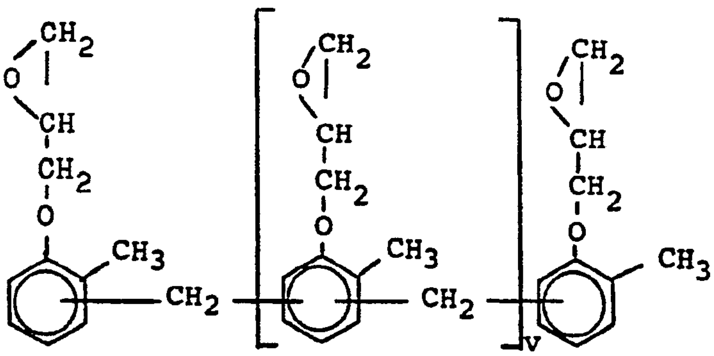

mit v= 1, 2, 3...

Die Verbindung der Formel IX müssen für die Umsetzung zu den erfindungsgemäßen Stabilisatoren nicht in reiner Form vorliegen, sondern können auch in Form der entsprechenden Epoxidharze technischer Qualität verwendet werden.

Die neuen Stabilisatoren lassen sich problemlos in die zu stabilisierenden Polymeren einarbeiten und sind hervorragend zum Stabilisieren derselben gegen den lichtinduzierten oxidativen Abbau, d.h. ihre Schädigung durch Einwirkung von Sauerstoff, Wärme und Licht, geeignet. Neben der ausgezeichneten Stabilisatorwirksamkeit zeichnet die neuen Stabilisatoren ihre sehr gute Verträglichkeit mit den zu stabilisierenden Polymeren aus.

Beispiele für erfolgreich zu stabilisierende Polymere sind :

Polymere, die sich von einfach oder doppelt ungesättigten Kohlenwasserstoffen ableiten, z.B. Polyolefine wie Polyethylen, das gegebenenfalls vernetzt sein kann, Polypropylen, Polybuten-1, Polyisobuten, Polymethylbuten-1, Polymethylpenten-1, Polyisopren, Polybutadien, Polystyrol, Copolymere der den genannten Homopolymeren zugrundeliegenden Monomeren, wie Ethylen-Propylen-Copolymere, Propylen-Buten-1-Copolymere, Propylen-Isobuten-Copolymere, Styrol-Butadien-Copolymere sowie Terpolymere von Ethylen und Propylen mit einem Dien, wie z.B. Hexadien, Dicyclopentadien oder Ethylidennorbornen ; Mischungen der obengenannten Homopolymeren, wie beispielsweise Gemische von Polypropylen und Polyethylen, Polypropylen und Polybuten-1, Polypropylen und Polyisobuten oder von Butadien-Acrylnitril-Copolymerisaten mit einem Styrol-Butadien-Copolymerisat.

Halogenhaltige Vinylpolymere, wie Polyvinylchlorid, Polyvinylidenchlorid, Polyvinylfluorid, Polychloropren, Chlorkautschuke sowie Copolymere von Vinylchlorid und Vinylidenchlorid untereinander und mit anderen olefinisch ungesättigten Monomeren.

Polymere, die sich von α, β-ungesättigten Säuren und deren Derivaten ableiten, wie Polyacrylate und Polymethacrylate, Polyacrylamide und Polyacrylnitril sowie deren Copolymere untereinander und mit anderen Vinylverbindungen, wie Acrylnitril-Butadien-Styrol-, Acrylnitril-Styrol- und Acrylnitril-Styrol-Acrylester-Copolymerisate.

Polymere, die sich von ungesättigten Alkoholen und Aminen bzw. deren Acrylderivaten oder Acetalen ableiten, wie Polyvinylalkohol, Polyvinylacetat, -stearat, -benzoat, -maleat, Polyvinylbutyral, Polyallylphthalat, Polyallylmelamin und deren Copolymere mit anderen Vinylverbindungen, wie Ethylen-Vinylverbindungen, wie EthylenVinylacetat-Copolymere. Homo- und Copolymere, die sich von Epoxiden ableiten, wie Polyethylenoxid oder die Polymerisate, die sich von Bisglycidylethern ableiten.

Polyacetale, wie Polyoximethylen und Polyoxiethylen sowie solche Polyoximethylene, die als Comonomeres Ethylenoxid enthalten.

Polyurethane und Polyharnstoffe

Polycarbonat

Polyamide und Copolyamide, die sich von Diaminen und Dicarbonsäuren und/oder von Aminocarbonsäuren oder den entsprechenden Lactamen ableiten, wie Polyamid 6, Polyamid 6/6, Polyamid 6/10, Polyamid 11, Polyamid 12.

Polyester, die sich von Dicarbonsäuren und Dialkoholen und/oder von Hydroxicarbonsäuren oder den entsprechenden Lactonen ableiten, wie Polyethylenterephthalat, Polybutylenterephthalat, Poly-1,4-dimethylol-cyclohexanterephthalat.

Vernetzte Polymerisate, die sich von Aldehyden einerseits und Phenolen, Harnstoffen und Melaminen andererseits ableiten, wie Phenol-Formaldehyd-, Harnstoff-Formaldehyd- und Melamin-Formaldehydharze.

Die neuen Verbindungen lassen sich schließlich auch als Stabilisatoren auf dem Harz- und Lacksektor einsetzen. Beispiele sind duroplastische und thermoplastische Acrylharze, welche für Autolackierungen verwendet werden, Acrylharzlacke, das sind die üblichen Einbrennlacke sowie ganz besonders Mischungen auf der Basis von heiß-vernetzbarem Acrylharz und Styrol sowie Lacke und Beschichtungen auf der Basis von Acryl-Melaminharz und Alkyd/Acryl/Melaminharz. Derartige Lacke können als weitere Zusatzstoffe andere übliche Lichtschutzmittel, phenolische Antioxidantien, Pigmente, Farbstoffe, Metalldesaktivatoren etc. enthalten.

Von besonderer Bedeutung ist die Stabilisierung von Polyolefinen, Styrolpolymerisaten, Polyamiden, Poly-(meth)-acrylaten und von Polyurethanen, für die sich die Verbindungen bevorzugt eignen. Beispiele hierfür sind Polyethylen hoher und niedriger Dichte, Polypropylen, Ethylen-Propylen-Copolymerisate, Polystyrol, Styrol-Butadien-Acrylnitril-Terpolymerisate ; Mischungen von Polyolefinen oder von Styrolpolymerisaten sowie Polyurethane auf Polyether- oder Polyesterbasis.

Die neuen Stabilisatoren werden nach allgemein üblichen Methoden in die Polymermassen eingearbeitet. Die Einarbeitung kann beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere direkt oder Einmischen in eine Lösung, Suspension oder Emulsion desselben, gegebenenfalls unter nachträglichem Verdunstenlassen des Lösungsmittels, erfolgen. Die Mengen liegen bei 0,01 bis 5, vorzugsweise bei 0,05 bis 2,5 und insbesondere bei 0,1 bis 1,0 Gew.-%, bezogen auf das zu stabilisierende Material. Die neuen Verbindungen können auch in Form eines Masterbatches, der diese Verbindungen beispielsweise in einer Konzentration von 1 bis 50, vorzugsweise 2,5 bis 20 Gew.-% enthält, den zu stabilisierenden Kunststoffen zugesetzt werden.

Die durch den Zusatz der erfindungsgemäßen Substanzen stabilisierten Kunststoffe können gegebenenfalls noch andere bekannte und übliche Zusätze, wie beispielsweise Antioxidantien auf Phenol- und Sulfidbasis, Metalldesaktivatoren und Lichtschutzmittel, Phosphitstabilisatoren, Metallverbindungen, Epoxidstabilisatoren und mehrwertige Alkohole enthalten.

Beispiele für Antioxidantien sind sterisch gehinderte Phenole wie 2,6-Di-tert.-butyl-4-methylphenol, 4,4′-Butyliden-bis-(2,6-di-tert.-butylphenol), 4,4′-Thio-bis-(2-tert.-butyl-5-methylphenol), 2,5-Di-tert.-butyl-4-hydroxianisol, 2,2-Bis-(3,5-di-tert.-butyl)-2-hydroxibenzyl)-malonsäuredioctadecylester, 1,3,5-Tris-(3,5-di-tert.-butyl-4-hydroxibenzyl)-2,4,6-trimethylbenzol, 2,4,6-Tri-(3,5-di-tert.-butyl-4-hydroxibenzyl)-phenol, phenolische Triazinverbindung wie 1,3,5-Tris-(3,5-di-tert.-butyl-4-hydroxibenzyl)-isocyanurat, Amide oder Ester der β-(3,5-Di-tert.-butyl-4-hydroxiphenyl)-propionsäure mit z.B. Octadecanol, 1,6-Hexandiol, 2,2′-Thiodiethylenglykol, Pentaerythrit und Tris-hydroxiethylisocyanurat, Hexamethylendiamin, Ester der 3,3-Bis-(3-tert.-butyl-4-hydroxiphenyl)-butansäuren mit z.B. Ethylenglykol, Thiodipropionsäureester mit Fettalkoholen, Ca- oder Ni-Salze des 3,5-Di-tert.-butyl-4-hydroxibenzylphosphonsäureethylesters, Dioctadecylsulfid und - disulfid.

Beispiele für Metalldesaktivatoren sind N,N′-Diphenyloxalsäurediamid, N-Salizylal-N′-salizyloylhydrazin, N,N′-Bis-salizyloylhydrazin, N,N′-Bis-(5,5-di-tert.-butyl-4-hydroxyphenylpropionyl)-hydrazin, 3-Salizyloylamino-1,2,4-triazol, Bis-benzyliden-Oxalsäuredihydrazid, Tris [2-tert.-butyl-4-thio (2′-methyl-4′-hydroxy-5′-tert.-butyl) phenyl-5-methyl] phenylphosphit, 2,2′-Oxamido-bis-[ethyl-3-(3,5-di-tert.-butyl-4-hydroxiphenyl)propionat].

Zu den UV-Absorbern und Lichtschutzmitteln gehören 2-(2′-Hydroxiphenyl)-benztriazole wie z.B. das 5-Chlor-3′,5′-di-tert.-butyl- und 5-Chlor-3′,5′-di-tert.-amyl-Derivat, 2-Hydroxibenzophenone wie z.B. das 4-Heptoxi-, 4-Octoxi- oder 4-Dodecycloxi-Derivat, Salizylate wie Octylphenylsalizylat, Nickelkomplexe wie z.B. der Komplex mit 2,2′-Thio-bis-4-(1,1,3,3-tetramethylbutyl)-phenol und Butylamin oder anderen Aminen, oder mit 2-Hydroxi-4-octoxibenzophenon, mit Dialkyldithiocarbaminsäuren bzw. Dialkyldithiophosphonsäuren, Oxalsäurediamide und sterisch gehinderte Amine, z.B. Bis (2,2,6,6-tetramethyl-4-piperidinyl) sebacat, Polyester der Bernsteinsäure mit N-(2-hydroxyethyl)-2,2,6,6-tetramethyl-4-Hydroxi-piperidin, N,N'-Bis (2,2,6,6-tetramethyl-4-piperidinyl) hexamethylen-diamin, 2,2,4,4-Tetramethyl-7-oxa-3,20 -diaza-21-oxo-dispiro

[5,1,11,2]-heneicosan, 1,1'-(1,2-ethandiyl)bis-(3,3,5,5-tetramethylpiperazinon), Kondensationsprodukt von N,N'-Bis (2,2,6,6-tetramethyl-4-piperidyl) hexamethylen-diamin mit Dibromethan oder mit 4-tert-0ctylamino2,6-dichlor-1,3,5-triazin oder mit 4-(N-Morpholinyl)-2,6-dichlor-1,3,5-triazin, Tetrakris-(2,2-tetramethyl-4piperidyl-1,2,3,4-butantetracarbonsäure.

Als Phosphite sind aliphatische, aromatische oder aliphatisch-aromatische wie z.B. Trisnonylphenylphosphit, Tris-(2,4-di-tert-butylphenyl)-phosphit, Tris-(2-tert-butylphenyl)-phosphit oder auch Ester des Pentaerythritphosphits zu nennen

Als Stabilisatoren bekannte Metallverbindungen sind z.B. : Calcium, Barium-, Strontium-, Zink-, Cadmium, Magnesium-, Aluminium-und Bleiseifen aliphatischer Carbonsäuren oder Oxicarbonsäuren mit etwa 12 bis 32 C-Atomen, Salze der genannten Metalle mit aromatischen Carobnsäuren wie Benzoate oder Salizylate sowie (Alkyl-)-Phenolate dieser Metalle, ferner Organozinnverbindungen, wie z.B. Dialkylzinnthioglykolate und -carboxilate, sowie Metalloxide, z.B. Oxide des Calcium, Magnesium Zink, Aluminium oder des Silicium.

Bekannte Epoxistabilisatoren sind z.B. epoxidierte höhere Fettsäuren wie epoxidiertes Sojabohnenöl, Tallöl, Leinöl oder epoxidiertes Butyloleat sowie Epoxide langkettiger Olefine und Polyetherepoxide.

Mehrwertige Alkohole können beispielsweise Pentaerythrit, Trimethylolpropan, Sorbit oder Mannit sein, d.h. bevorzugt Alkohole mit 5 oder 6 C-Atomen und 2 bis 6 OH-Gruppen.

Eine wirksame Stabilisatorkombination für Poly-α-Olefine ; wie z.B. Hoch-, Mittel-und Niederdruckpolymerisate von $C_2$-bis $C_4$-α-Olefinen, insbesondere Polyethylen und Polypropylen oder von Copolymerisaten derartiger α-Olefine besteht auf 100 Gewichtsteile Polymer, beispielsweise aus 0,01 bis 5 Gewichtsteilen einer der erfindungsgemäß zu verwendenden Verbindungen, 0,05 bis 5 Gewichtsteilen eines phenolischen Stabilisators gegebenenfalls 0,01 bis 5 Gewichtseilen eines schwefelhaltigen Costabilisators sowie gegebenenfalls 0,01 bis 3 Gewichtsteilen einer basischen oder neutralen Metallseife, wie z.B. Calciumstearat oder Zinkstearat oder der entsprechenden Oxide, gegebenenfalls 0,01 bis 5 Gewichtsteile eines Phosphit-oder Phosphonitstabilisators sowie gegebenenfalls 0,01 bis 5 Gewichsteilen eines bekannten UV-Stabilisators aus der Gruppe der Alkoxihydroxibenzophenone, 4-Hydroxiphenylbenzotriazole, Benzylidenmalonsäuremononitrilester oder der sog. Quencher, wie z.B. Nickelchelate. Als sonstige übliche Zusätze sind z.B. Weichmacher, Gleitmittel, Emulgatoren, Füllstoffe, wie z.B. Kreide, Talkum, Asbest, Pigmente, optische Aufheller, Flammschutzmittel und Antistatika anzusehen.

Die erfindungsgemäß stabilisierten Kunststoffe können in verschiedenster Form angewendet werden, z.B. als Folien Fasern, Bändchen, Profile oder als Bindemittel für Lacke, Klebemittel oder Kitte.

Der weiteren Erläuterung der Erfindung dienen nachstehende Beispiele.

Beispiel 1

In einer 1-l-Rührapparatur mit Rückflußkühler, Innenthermometer und Tropftrichter wurden 72,8 g (0,02 mol) der Verbindung 30 in 300 ml Toluol vorgelegt und 1 g Triethylbenzylammoniumchlorid, 18,O g (0,23 mol) 50%ige Natronlauge und 77,2 g (0,40 Epoxidäquivalente) eines technishcen Bisphenol-A-Epoxids - (Verbindung 82) zugegeben. Das Reaktionsgemisch wurde auf 90°C aufgeheizt und 4 h bei dieser Temperatur kräftig gerührt. Nach beendigung der Umsetzung wurde 3mal mit je 150 ml Wasser ausgeschüttelt, die organische Phase über Natriumsulfat getrocknet und nach dem Filtrieren das Toluol abdestilliert. Dann wurde der Rückstand bei ca.$_{1,3\ mbar}$ 4 h lang auf 200°C erhitzt. Man erhielt 139,5 g eines sehr harten glasartigen Feststoffes mit einem Erweichungspunkt > 250°C und einer mittleren Molmasse > 5000.

Beispiel 2

Es wurde das Zwischenprodukt gemäß Beispiel 1 hergestellt, welches dann jedoch 4 h auf 150°C erhitzt wurde. Das erhaltene spröde, glasartige produkt hatte einen Erweichungspunkt von 220°C und eine mittlere Molmasse von ca. 5000.

Beispiel 3

Das Beispiel 2 wurde wiederholt, jedoch wurde das Zwischenprodukt nur 2 h erhitzt. Das erhaltene

spröde. glasartige Produkt hatte einen Erweichungspunkt von 175°C und eine mittlere Molmasse von 3300.

Beispiel 4

Analog Beispiel 1 wurden 72,8 g (0,2 mol) der Verbindung 30 mit 57,9 g (0,30 Epoxidäquivalente) eines technischen Epoxides auf Basis Bisphenol-A (Verbindung 82) umgesetzt. Das Zwischenprodukt wurde bei ca.$_{1,3\ mbar}$ 4 h auf 180°C erhitzt. Man erhielt 120,5 g eines fast farblosen, spröden glasartigen Produktes mit einem Erweichungspunkt von 168°C und einer mittleren Molmasse von 3000.

Beispiel 5

Es wurde wie in Beispiel 4 verfahren. wobei jedoch nur 54.0 g (0,28 Epoxidäquivalente) des BisphenolA-Epoxids verwendet wurden. Das spröde, amorphe Produkt (122,4 g) hatte einenErweichungspunkt von ca. 150°C und eine mittlere Molmasse von 2800.

Beispiel 6

Analog Beispiel 1 wurden 36,4 g (0,1 mol) der Verbindung 30 in 150 ml Toluol mit 23,2 g (0,12 Epoxidäquivalente) eines technischen Bisphenol-A-Epoxides (Verbindung 82) in Gegenwart von 0,5 g Triethylbenzylammoniumchlorid und 9,0 g (O,11 mol) 50%iger Natronlauge umgesetzt. Das ZwiSchenprodukt wurde dann bei ca.$_{1,3\ mbar}$ 4 h auf 180°C erhitzt. Man erhielt 56,3 g eines farblosen, amorphen Feststoffes mit einem Erweichungspunkt von 135°C und einer mittleren Molmasse von 1960.

Beispiel 7

Analog Beispiel 4 wurden 56,0 g (0,2 mol) der Verbindung 29 anstelle der Verbindung 30 umgesetzt. Man erhielt 105,3 g farbloses Produkt mit einem Erweichungspunkt > 250°C und einer Molmasse von 6800.

Beispiel 8

Analog Beispiel 1 wurden anbelle der Verbindung 30 28,0 g (0,1 mol) der Verbindung 29 mit 27,0 g -(0.14 Epoxidäquivalente) eines technischen Bisphenol-A-Epoxides (Verbindung 82) umgesetzt. Das Zwischenprodukt wurde 4 h bei ca.$_{1,3\ mbar}$ auf 180°C erhitzt. Es wurden 53,7 g farbloses Produkt mit einem Erweichungspunkt von 147°C und einer mittleren Molmasse von 2400 erhalten.

Beispiel 9

Analog Beispiel 6 wurden anstelle der Verbindung 30 28,0 g (0,1 mol) der Verbindung 29 umgesetzt. Dabei wurden 50,5 g farbloses, amorphes Produkt mit einem Erweichungspunkt von 130°C und einer mittleren Molmasse von 1640 erhalten.

Beispiel 10

Analog Beispiel 4 wurden anstelle der Verbindung 30 48,0 g (0,2 mol) der Verbindung 31 umgesetzt. Man erhielt 97,8 g farbloses amorphes Produkt mit einem ErweiBichungspunkt von 145°C und einer mittleren Molmasse von 2580.

Beispiel 11

In einer 1-l-Rührapparatur mit Innenthermometer, Rückflußkühler und Tropftrichter wurden 100 ml Toluol vorgelegt, 24,0 g (0,1 mol) der Verbindung 43 (Edukt I), 0,3 g Triethylbenzylammoniumchlorid. 9.0 g (0,11 mol) 50%ige Natronlauge und 38.6 g (0.20 Epoxidäquivalente) eines technischen Bisphenol-A-Epoxides (Verbindung 82 ; Edukt II) zugegeben und das Gemisch auf 90°C aufgeheizt und 4 h gerührt. Danach wurde 3mal mit je 50 ml Wasser ausgeschüttelt. die organische Phase mit Natriumsulfat getrocknet und nach der Filtration das Toluol abdestilliert. Das so erhaltene Zwischenprodukt wurde bei ca. 1,3 mbar 4 h auf 180°C erhitzt. Dabei wurden 82,3 g eines farblosen. sehr harten Produktes mit einem Erweichungspunkt 250°C erhalten.

Beispiel 12 bis 15

Die Durchführung erfolgte gemäß dem Beispiel 11.

| Bsp. | Edukt I g / Mol | | Edukt II g / Epoxid-äquivalente | | Produkt g | Ep(°C) | M |
|---|---|---|---|---|---|---|---|
| 12 | 24,0 | 0,1 | 34,8 | 0,18 | 50,3 | 235 | 6340 |
| 13 | " | " | 30,9 | 0,16 | 47,3 | 150 | 3730 |
| 14 | " | " | 27,1 | 0,14 | 45,2 | 132 | 1820 |
| 15 | " | " | 23,2 | 0,12 | 42,6 | 125 | 1330 |

Beispiele 16 bis 18

Analog Beispiel 11 wurden anstatt der Verbindung 43 27.7 g (0,1 mol) der Verbindung 72 als Edukt I sowie 17.6 g (0,22 mol) 50%ige Natronlauge eingesetzt

| Bsp. | Edukt I g / Mol | | Edukt II g / Epoxid-äquivalente | | Produkt g | Ep(°C) | M |
|---|---|---|---|---|---|---|---|
| 16 | 27,7 | 0,1 | 26,9 | 0,14 | 48,9 | 137 | 2200 |
| 17 | " | " | 29,3 | 0,15 | 50,7 | 145 | 3300 |
| 18 | " | " | 31,2 | 0,16 | 52,5 | 156 | 5000 |

Beispiel 19

Die Durchführung erfolgte wie im Beispiel 16, wobei hier jedoch kein Triethylbenzylammoniumchlorid zugesetzt wurde. Es wurden 46,0 g amorphes Produkt mit einem Erweichungspunkt von 138°C und einer mittleren Molmasse von 2100 erhalten.

Beispiel 20

Es wurde analog beispiel 19 verfahren, wobei die Edukte jedoch unter Rückfluß miteinander umgesetzt wurden. Man erhielt 47,3 g amorphes Produkt mit einem Erweichungspunkt von 139°C und einer mittleren Molmasse von 2400.

Beispiel 21

In einem 1 -l-Rührgefäß mit Innenthermometer und Rückflußkühler wurden 200 ml Toluol vorgelegt, 55,3 g (0,2 mol) der Verbindung 72 (Edukt I) 0,6 g Benzyltriethylammoniumchlorid und 19,8 g KOH-Pulver zugegeben und der Ansatz auf 90°C aufgeheizt. Dann wurden 52,5 g (0,30 Epoxidäquivalente) eines technischen Bisphenol-F-Epoxids (Verbindung 81, Edukt II) eingetragen und der Ansatz 2 h bei 90°C weitergerührt. Anschließend wurde zweimal druckfiltriert, das Toluol abdestilliert und das so erhaltene Zwischenprodukt bei $_{40\ mbar}$3 h auf 180°C aufgeheizt. Man erhielt 97,3 g amorphes, nahezu farbloses Produkt mit einem Erweichungspunkt von 125°C und einer mittleren Molmasse von 2300.

Beispiel 22

Analog zu Beispiel 21 wurden 55,3 g (0,2 mol) der Verbindung 72 (Edukt I) mit 37,5 g (0,30 Epoxidäquivalente) eines technischen Butandioldiglycidylethers (Verbindung 77 ; Edukt II) umgesetzt. Man erhielt 79,8 g farbloses, amorphes Produkt mit einem Erweichungspunkt von 59°C und einer mittleren Molmasse von 1720.

Beispiel 23

In einer 500-ml-Rührapparatur mit Rückflußkühler und Innenthermometer wurden 200 ml Toluol, 55,3 (0,2 mol) der Verbindung 72, 19,8 g KOH-Pulver und 53,8 g (0,28 Epoxidäquivalente) eines technischen Bisphenol-A-Epoxids (Verbindung 82) zusammengegeben und auf 90°C aufgeheizt, 1 h gerührt, zweimal druckfiltriert und das Toluol ab destilliert. Das so erhaltene Zwischenprodukt wurde bei $_{40\ mbar}$3 h auf 180°C erhitzt. Man erhielt 96,6 g farbloses, amorphes Produkt mit einem Erweichungspunkt von 132°C und einer mittleren Molmasse von 2200.

Beispiel 24

Dieses Beispiel zeigt die Flüchtigkeit der erfindungsgemäßen Stabilisatoren im Vergleich zu Stabilisatoren der DE-PS 26 06 026 und DE-OS 26 34 957.

Die Flüchtigkeiten wurden in einer Apparatur zur thermogravimetrischen Analyse bestimmt. Gleiche Mengen (500 mg) der erfindungsgemäßen Verbindungen bzw. der Vergleichssubstanzen wurden dazu im Stickstoffstrom (1 l/min) mit einer Aufheizgeschwindigkeit von 2 K/min bis auf 300°C erhitzt und der Substanzverlust in Gewichtsprozent gemessen.

| Stabilisator gem. Beispiel | Substanzverlust in Gew.-% beim Erreichen ... °C | | | |
|---|---|---|---|---|
| | 200 | 260 | 300 | 10 min bei 300 |
| 4 | 0 | 0,1 | 0,6 | 2,5 |
| 6 | 0,1 | 0,2 | 0,9 | 2,7 |
| 8 | 0 | 0,1 | 0,6 | 2,0 |
| 10 | 0 | 0,2 | 1,0 | 2,7 |
| 17 | 0 | 0 | 3,3 | 8,5 |
| 20 | 0 | 0,2 | 2,6 | 8,3 |
| 22 | 0,1 | 0,5 | 4,2 | 9,4 |
| Vergleich A[1)] | 0,8 | 2,8 | 12,3 | 29,8 |
| Vergleich B[2)] | 17,9 | 75 | bei 280°C: 100 % | |

1) Stabilisator gemäß Beispiel 31 der DE-PS 26 06 026

2) Stabilisator gemäß Beispiel 21 der DE-OS 26 34 957

Beispiele 25 und 26

Beispiel 25

Eine Mischung bestehend aus

| | | |
|---|---|---|
| 100 | Gew.-T. | Polypropylen (MFI 230/5: 4 g/min; Dichte bei 23°C: 0,903 g/cm³), |
| 0,1 | " | Bis-[3,3-bis-(4'-hydroxy-3'-tert.-butylphenyl)-butansäure]-glykolester, |
| 0,1 | " | Calciumstearat und |
| 0,3 | " | des zu prüfenden erfindungsgemäßen Stabilisators |

wurde in einem Laborschnellmischer homogenisiert. Diese Mischung wurde in einer Windsor-Spritzgußmaschine der Type SP 50 bei 240°C zu 60x60x1-mm-Platten verspritzt. Aus diesen Platten wurden T-förmige Prüfkörper nach DIN 53 383 ausgestanzt.

Zur Bestimmung der Wärmealterungsbeständigkeit wurden diese Prüfkörper in einem Umlufttrockenschrank in ein motorgetriebenes Gestell mit rotierenden Horden eingehängt und bei gleichmäßiger Frischluftzufuhr einer Temperaturbelastung von 140°C unterworfen.

Die Zeit, nach der an einigen Stellen eine beginnende lokale Versprödung auftrat, nach DIN 53 383 gekennzeichnet durch Bildung verfärbter, trüber, teilweise abbröckelnder Stellen, wurde festgehalten.

Die Ergebnisse zeigt folgende Tabelle :

| Stabilisator gemäß Beispiel | beginnende Versprödung nach ... Tagen |
|---|---|
| 20 | 31 |
| Vergleich A | 13 |
| Vergleich C[1)] | 5 |

1) ohne zu prüfenden Stabilisator

Beispiel 26

Aus einer Mischung bestehend aus

| | | |
|---|---|---|
| 100 | Gew.-T | Polypropylenpulver (MFI 230/5: 18 g/10 min; Dichte bei 23°C; 0,903 g/cm³); |
| 0,1 | " | Bis-[3,3-bis(4'-hydroxy-3'-tert.-butylphenyl)-butansäure]-glykolester |
| 0,1 | " | Calciumstearat, |
| 0,05 | " | Tri-(2,4-di-tert.-butylphenyl)-phosphit und |
| 0,3 | " | des zu prüfenden Stabilisators |

wurden wie in Beispiel 25 Prüfkörper hergestellt und der Wärmealterungsbeständigkeitsprüfung unterworfen. Die Ergebnisse zeigt folgende Tabelle :

| Stabilisator gemäß Beispiel | beginnende Versprödung nach ... Tagen |
|---|---|
| 14 | 39 |
| 20 | 37 |
| Vergleich A | 21 |
| Vergleich C | 20 |
| Vergleich D[1] | 6 |

1) unstabilisiertes Polypropylen

Beispiel 27

Eine Mischung bestehend aus

| | | |
|---|---|---|
| 100 | Gew.-T | Polypropylen (MFI 230/5: 4 g/10 min, Dichte bei 23°C 0,903 g/cm³); |
| 0,1 | " | Bis-[3,3-bis-(4'-hydroxy-3'-tert.-butylphenyl)-butansäure]-glykolester, |
| 0,1 | " | Calciumstearat und |
| 0,15 | " | des zu prüfenden Stabilisators |

wurde in einem Laborschnellmischer homogenisiert. Diese so hergestellte stabilisierte Mischung wurde auf einer Labor-Folienblasanlage (Schneckendurchmesser 30 mm, Länge 20 D) zu Blasfolien von ca. 100 µm Dicke verarbeitet. Aus diesen Folien wurden Prüfkörper nach DIN 53 455, Form 3, verkleinert im Maßstab 1 : 3, ausgestanzt.

Zur Bestimmung der Lichtstabilität wurden diese Proben in einer Anordnung gemäß DIN 53 387 Nr. 5.1 Anmerkung 2 in einem Schnellbelichtungs- und Bewitterungsgerät ®Xenotest 450 (Original Hanau Quarzlampen GmbH) der Bestrahlung mit Wechsellicht unterworfen. Die Strahlungsintensität wurde durch 6 IR-Filter + 1 UV-Filter moduliert. Die Lichtbeständigkeit wurde nach DIN 53 387 (17 min Trockenperiode, 3 min Beregnung, Schwarztafeltemperatur 45 ± 5°C, relative Luftfeuchtigkeit während der Trockenperiode 70 bis 75%) geprüft. Gemessen wurde die Reißdehnung auf einer Zugprüfmaschine bei einer Abzugsgeschwindigkeit von 5 cm/min nach einer bestimmten Belichtungszeit in Stunden.

Die Ergebnisse sind in folgender Tabelle zusammengestellt.

| Stabilisator gemäß Beispiel | Belichtungszeit bis zum 50-%-Wert (Reißdehnung bez. auf Ausgangswert) | 10-%-Wert |
|---|---|---|
| 14 | 700 h | 790 h |
| Vergleich C | 220 h | 260 h |

Beispiel 28

Aus den gemäß Beispiel 25 hergestellten Platten wurden Prüfkörper nach DIN 53 455, Form 3, verkleinert im Maßstab 1 : 3 ausgestanzt.

Zur Bestimmung der Lichtstabilität wurden diese Proben im Gerät Xenotest 450 der Bestrahlung mit Dauerlicht unterworfen. Verwendet wurden 6 IR- und 1 UV-Filter sowie eine Schwarztafeltemperatur von ca. 75°C (am Ende der Trockenperiode), 17 min Trockenperiode und 3 min Beregnung und eine relative Luftfeuchtigkeit während der Trockenperiode von ca. 70%. Gemessen wurde die Reißdehnung auf einer Zugprüfmaschine bei einer Abzugsgeschwindigkeit von 5 cm/min nach bestimmten Belichtungszeiten. Aus diesen Daten wurden dann folgende Ergebnisse ermittelt :

| Stabilisator gemäß Beispiel | Belichtungszeit bis zum 50-%-Wert | 10-%-Wert |
|---|---|---|
| | (Reißdehnung bez. auf Ausgangswert) | |
| 20 | 1600 h | 1750 h |
| Vergleich C | 100 h | 175 h |

**Ansprüche**

**Patentansprüche für die Vertragsstaaten : BE CH DE FR GB IT LI NL SE**

1. Verbindungen der Formel (I)

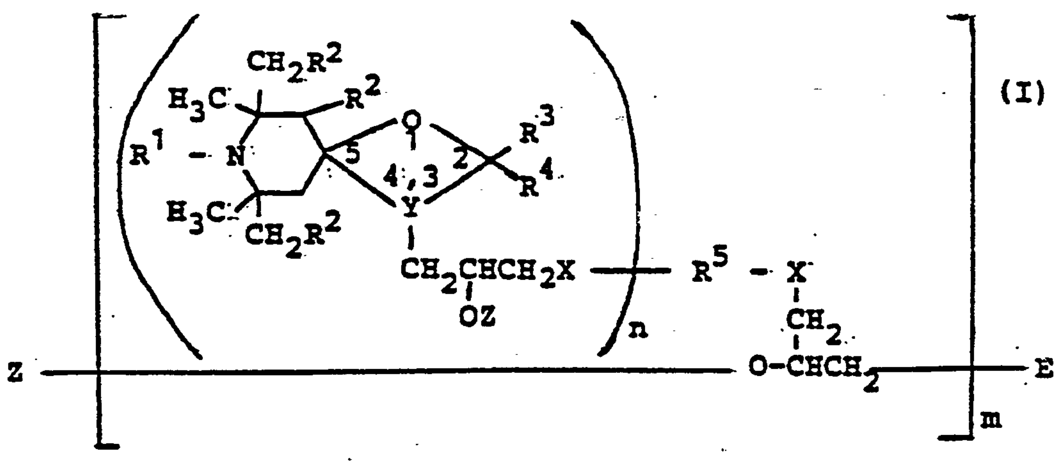

(I)

worin

n eine ganze Zahl von 1 bis 4 ist,

m eine ganze Zahl von 2 bis 100 ist,

$R^1$ Wasserstoff, $C_1$-bis $C_4$-Alkyl, Benzyl, Allyl, $C_2$-bis $C_{30}$-Alkanoyl, $C_2$-bis $C_{20}$-Alkenoyl, $C_7$-bis $C_{11}$-Aroyl, $C_8$-bis $C_{14}$-Arylalkanoyl oder $C_8$-bis $C_{20}$-Alkylaryl ist,

$R^2$ Wasserstoff oder $C_1$-bis $C_4$-Alkyl ist,

$R^3$ und $R^4$ gleich oder verschieden sind und Wasserstoff, $C_1$-bis $C_{13}$-Alkyl, unsubstituiertes oder durch Chlor oder $C_1$-bis $C_4$-Alkyl substituiertes Phenyl, unsubstituiertes oder durch $C_1$-bis $C_4$-Alkyl substituiertes $C_7$-bis $C_{14}$-Aralkyl oder zusammen mit dem sie bindenden Kohlenstoffatom einen unsubstituierten oder durch bis zu vier $C_1$bis $C_4$-Alkylgruppen subbstituierten $C_5$-bis $C_{12}$-Cycloalkyl-oder Piperidinring bedeuten,

$R^5$ bei n = 1 $C_2$-bis $C_{18}$-Alkylen, unsubstituiertes oder durch bis zu zwei $C_1$-bis $C_4$-Alkylgruppen substituiertes Phenylen, α, ω-Dicarboxi-$C_1$-bis $C_8$-Alkylen, einen Dicarboxy-$C_6$-Ring, $C_7$-bis $C_{14}$-Aralkylen, Cycloalkylen, Dicycloalkylen, Tricycloalkylen, Bismethylenmonocycloalkylen, Bismethylendicycloalkylen,

Bismethylentricycloalkylen, Arylen, Bisarylenalkyl, wobei diese Reste auch durch Brom oder Chlor oder $C_1$- bis $C_4$-Alkyreste substituiert sein können, einen Rest

$$-R^6-O-[CH_2\underset{OZ}{\underset{|}{C}}HCH_2-O-R^6-O]_r$$

mit

r = 1 bis 20 und $R^6$ gleich $C_2$-bis $C_{12}$-Alkylen, Cycloalkylen, Dicycloalkylen, Tricycloalkylen, Bismethylenmonocycloalkylen, Bismethylendicycloalkylen, Bismethylentricycloalkylen, Arylen, Bisarylenalkyl, wobei diese Reste auch durch Brom oder Chlor bzw. $C_1$-bis $C_4$-Alkylreste substituiert sein können bedeutet,

$R^5$ bei n = 2 den Rest eines trifunktionellen Alkohols oder Amins, den trifunktionellen Rest eines aliphatischen Alkohols, der noch weitere Hydroxylgruppen enthält, den trifunktionellen Rest eines Novolaks auf Basis Phenol, Kresol, Bisphenol-F oder Bisphenol-A bedeutet,

$R^5$ bei n = 3 den tetrafunktionellen Rest eines aliphatischen Alkohols oder Amins oder eines Novolaks auf Basis Phenol, Kresol, Bisphenol-F oder Bisphenol-A oder den Rest

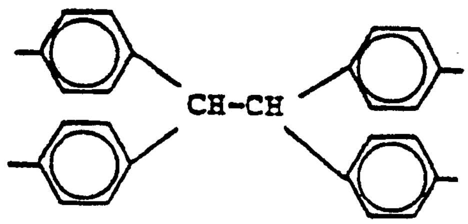

bedeutet

$R^5$ bei n = 4 einen pentafunktionellen Rest eines Polyols, eines Polyamins, eines Novolaks auf Basis Phenol, Kresol, Bisphenol-F oder Bisphenol-A bedeutet,

Y 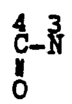 oder 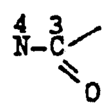 bedeutet und

die Ringpositionen 3, 4 in Formel (I) einnimmt,

X $-O-$ oder $-\underset{R^7}{\underset{|}{N}}-$ darstellt, wobei $R^7$

Wasserstoff, $C_1$-bis $C_{30}$-Alkyl, ein durch 1 bis 4 $C_1$-bis $C_4$-Alkylgruppen substituierter Piperidinring oder ein Rest der Formel (II)

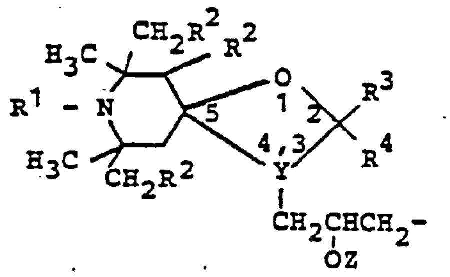

(II)

worin $R^1$, $R^2$, $R^3$, $R^4$, Y die oben angegebenen Bedeutungen haben, ist, Z Wasserstoff, ein Rest der Formel (III)

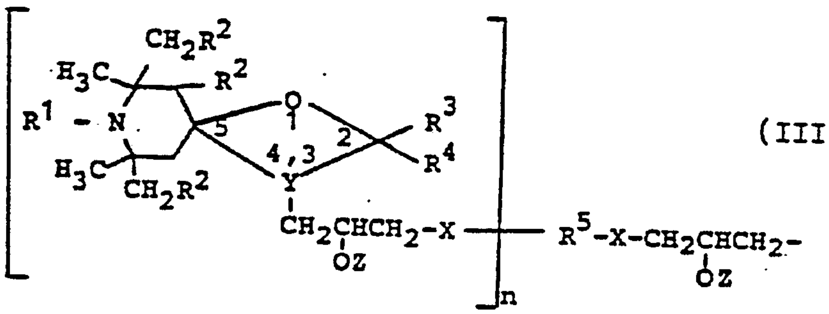

(III)

worin $R^1$, $R^2$, $R^3$, $R^4$, $R^5$, X, Y, Z und n die oben angegebenen Bedeutungen haben, oder ein Rest der Formel (II) ist,

E für Wasserstoff, Hydroxyl, einen Rest der Formel (IV), (V) oder (VI)

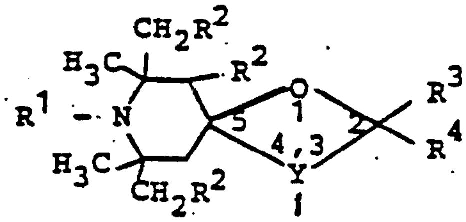

(IV)

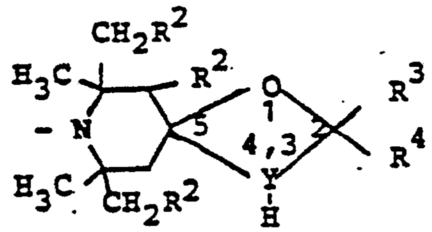

(V)

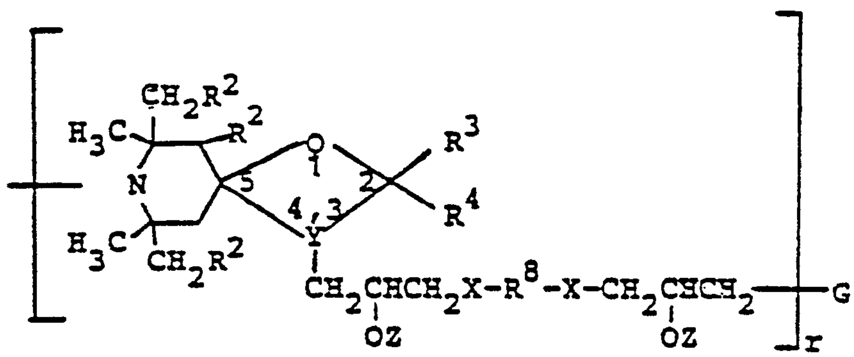

(VI)

worin $R^1$, $R^2$, $R^3$, $R^4$, $R^5$, X, Y und Z die oben angegebenen Bedeutungen haben, r eine ganze Zahl von 1 bis 100, darstellt und G ein Rest der Formel (IV) oder (V) ist und $R^8$ die Bedeutung der Formel (VII)

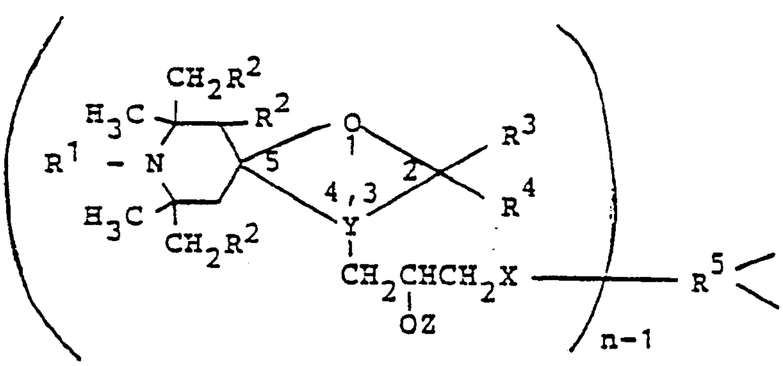

(VII)

worin $R^1$, $R^2$, $R^3$, $R^4$, $R^5$, X, Y, Z und n die oben angegebenen Bedeutungen haben, hat.

2. Verfahren zur Herstellung von Verbindungen der allgemeinen Formel (I) dadurch gekennzeichnet, daß Diazaoxaspirodecane der Formel (VIIIa) oder (VIIIb)

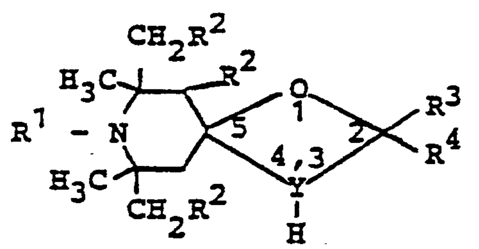

(VIIIa)

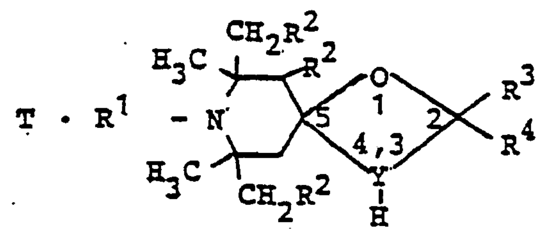

(VIIIb)

worin $R^1$, $R^2$, $R^3$, $R^4$ und Y die oben angegebenen Bedeutungen haben und T eine nicht oxidierende Mineralsäure oder eine aliphatische oder aromatische Sulfon-oder Phosphonsäure, eine aliphatische Mono-, Di- oder Polycarbonsäure oder eine aromatische Mono-oder Dicarbonsäure ist, und Epoxiden der Formel-(IXa) oder (IXb)

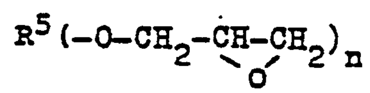

(IXa)

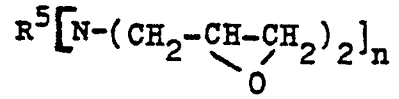

(IXb)

worin $R^5$ und n die oben angegebenen Bedeutungen haben, umgesetzt werden, wobei die Verbindungen-(VIIIa) und (VIIIb) in einem inerten organischen Lösungsmittel in Gegenwart der 0,2-bis 1,5fach äquivalenten Menge einer Base, bezogen auf die Verbindung (VIIIa), oder der 1,2-bis 2,5fach äquivalenten Menge einer Base bei Einsatz der Verbindung (VIIIb), bei einer Temperatur von 40 bis 180°C mit einem Unterschuß von einem bis zu einem fünftel Äquivalent, bezogen auf die Epoxidgruppen in den Verbindungen (IXa) oder-(IXb), eingesetzt werden, wobei gegebenenfalls ein Phasentransferkatalysator zugesetzt wird und die so hergestellten Zwischenprodukte anschließend in Substanz oder in Lösung, gegebenenfalls in Gegenwart von Katalysatoren, in höhermolekulare Verbindungen überführt werden

3. Verwendung der Verbindungen nach Anspruch 1 zum Stabilisieren von synthetischen Polymeren, in einer Menge von 0,01 bis 5 Gew.-%, bezogenaüf das zu stabilisierende Material.

4. Verwendung nach Anspruch 3, dadurch gekennzeichnet, daß das Polymere ein Polyolefin, ein Polyacrylat, ein Polymethacrylat, oder ein Homo-oder Copolymerisat des Styrols ist.

5. Verwendung nach Anspruch 3, dadurch gekennzeichnet, daß das Polymere den Feststoffanteil eines Lackes darstellt.

6. Verfahren zum Stabilisieren von synthetischen Polymeren gegen den schädigenden einfluß von Licht, darduch gekennzeichnet, daß man den Polymeren, gegebenenfalls neben bisher bekannten, stabilisierend wirkenden Stoffen 0,01 bis 5 Gew-%, bezogen auf Polymeres, eines Stabilisators nach Anspruch 1 zusetzt.

7. Gegen UV-Zersetzung stabilisierte, synthetische Polymere, in denen 0,01 bis 5 Gew-% bezogen auf Polymeres, eines Stabilisators nach Anspruch 1 enthalten sind.

**Patentansprüche für den Vertragsstaat : AT**

1. Verfahren zur Herstellung von Verbindungen der formel (I)

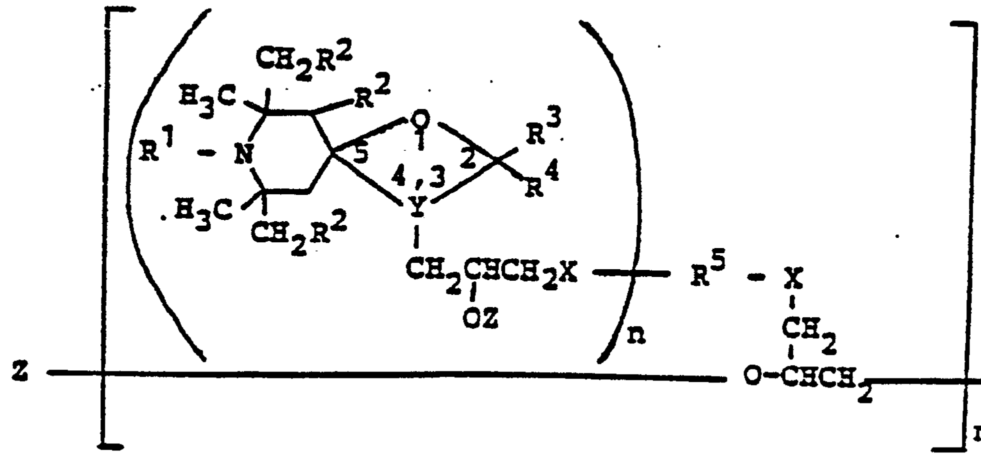

(I)

worin

n eine ganze Zahl von 1 bis 4 ist,

m eine ganze Zahl von 2 bis 100 ist,

$R^1$ Wasserstoff, $C_1$-bis $C_4$-Alkyl, Benzyl, Allyl, $C_2$-bis $C_{30}$-Alkanoyl, $C_2$-bis $C_{20}$-Alkenoyl, $C_7$-bis $C_{11}$-Aroyl, $C_8$-bis $C_{14}$-Arylalkanoyl oder $C_8$-bis $C_{20}$-Alkylaryl ist,

$R^2$ Wasserstoff oder $C_1$-bis $C_4$-Alkyl ist,

$R^3$ und $R^4$ gleicch oder verschieden sind und Wasserstoff, $C_1$-bis $C_{13}$-Alkyl, unsubstituiertes oder durch Chlor oder $C_1$-bis $C_4$-Alkyl substituiertes Phenyl, unsubstituiertes oder durch $C_1$-bis $C_4$-Alkyl substituiertes $C_7$-bis $C_{14}$-Aralkyl oder zusammen mit dem sie bindenden Kohlenstoffatom einen unsubstituierten oder durch bis zu vier $C_1$-bis $C_4$-Alkylgruppen substituierten $C_5$-bis $C_{12}$-Cycloalkyl-oder Piperidinring bedeuten,

$R^5$ bei n = 1 $C_2$-bis $C_{18}$-Alkylen. unsubstituiertes oder durch bis zu zwei $C_1$-bis $C_4$-Alkygruppen substituiertes Phenylen, α, ω-Dicarboxi-$C_1$-bis $C_8$-Alkylen, einen Dicarboxy-$C_6$-Ring, $C_7$-bis $C_{14}$-Aralkylen, Cycloalkylen, Dicyctoalkylen, Tricycloalkylen, Bismethylenmonocycloalkylen, Bismethylendicycloalkylen, Bismethylentricycloalkylen, Arylen, Bisarylenalkyl, wobei diese Reste auch durch Brom oder Chlor oder $C_1$-bis $C_4$-Alkylreste substituiert sein können, einen Rest

$$-R^6-O-[\underset{OZ}{CH_2\overset{|}{C}HCH_2}-O-R^6-O]_r$$

mit

r = 1 bis 20 und $R^6$ gleich $C_2$-bis $C_{12}$-Alkylen, Cycloalkylen, Dicycloalkylen, Tricycloalkylen, Bismethylenmonocycloalkylen, Bismethylendicycloalkylen, Bismethylentricycloalkylen, Arylen, Bisarylenalkyl, wobei diese Reste auch durch Brom oder Chlor bzw. $C_1$-bis $C_4$-Alkylreste substituiert sein können, bedeutet.

$R^5$ bei n = 2 den Rest eines trifunktionellen Alkohols oder Amins, den trifunktionellen Rest eines aliphatischen Alkohols, der noch weitere Hydroxylgruppen enthält, den trifunktionellen Rest eines Novolaks auf Basis Phenol, Kresol, Bisphenol-F oder Bisphenol-A bedeutet,

$R^5$ bei n = 3 den tetrafunktionellen Rest eines aliphatischen Alkohols oder Amins oder eines Novolaks

auf Basis Phenol, Kresol, Bisphenol-F oder Bisphenol-A oder den Rest

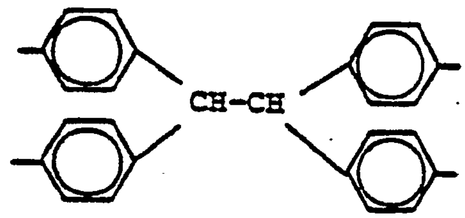

bedeutet,

$R^5$ bei n = 4 einen pentafunktionellen Rest eines Polyols, eines Polyamins, eines Novolaks auf Basis Phenol, Kresol, Bisphenol-F oder Bisphenol-A bedeutet,

Y 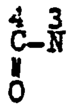 oder 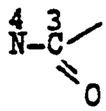 bedeutet und

die Ringpositionen 3, 4 in Formel (I) einnimmt,

X -O- oder 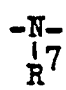 darstellt, wobei $R^7$

Wasserstoff, $C_1$bis $C_{30}$-Alkyl, ein durch 1 bis 4 $C_1$-bis $C_4$-Alkylgruppen substituierter Piperidinring oder ein Rest der Formel (II)

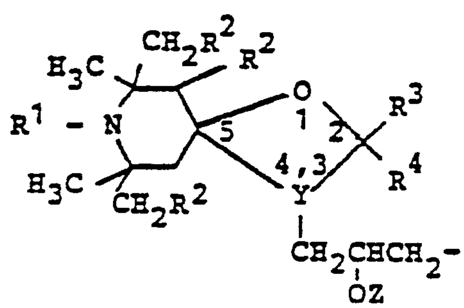

(II)

worin $R^1$, $R^2$, $R^3$, $R^4$, Y die oben angegebenen Bedeutungen haben, ist, Z Wasserstoff, ein Rest der Formel (III)

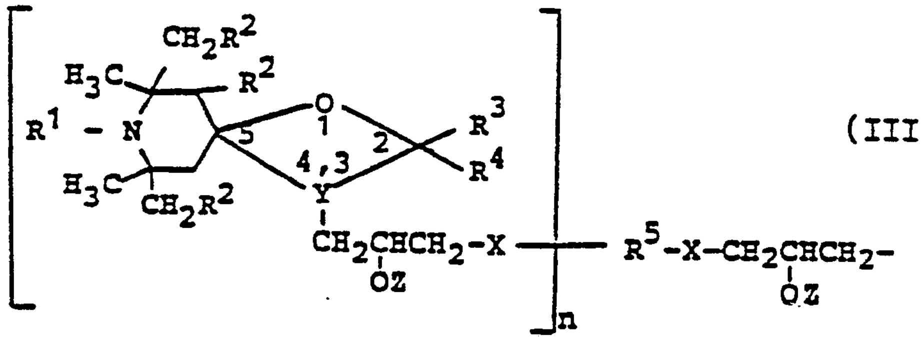

(III)

worin $R^1$, $R^2$, $R^3$, $R^4$, $R^5$, X, Y, Z und n die oben angegebenen Bedeutungen haben, oder ein Rest der Formel (II) ist,

E für Wasserstoff, Hydroxyl, einen Rest der Formel (IV), (V) oder (VI)

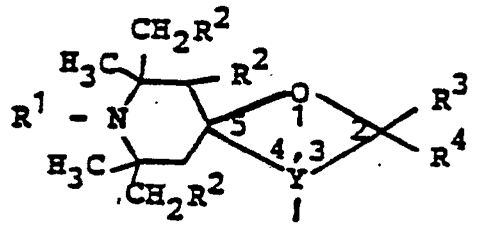

(IV)

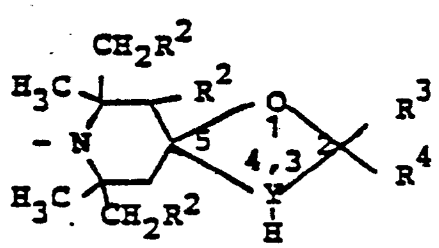

(V)

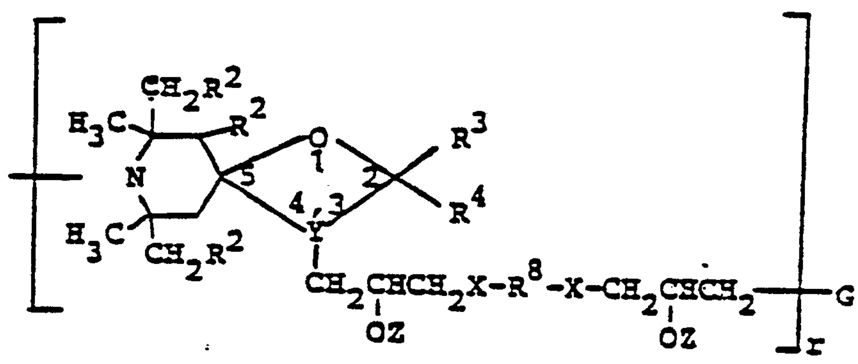

(VI)

worin $R^1$, $R^2$, $R^3$, $R^4$, $R^5$, X, Y, und Z die oben angegebenen Bedeutungen haben, r eine ganze Zhal von 1 bis 100 darstellt und G ein Rest der Formel (IV) oder (V) ist und $R^8$ die Bedeutung der Formel (VII)

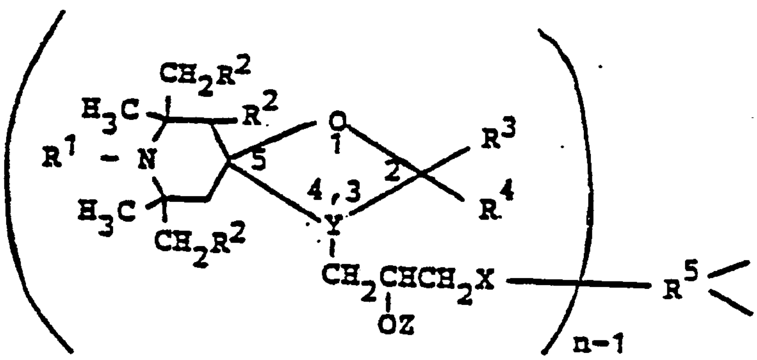

(VII)

worin $R^1$, $R^2$, $R^3$, $R^4$, $R^5$, X, Y, Z und n die oben angegebenen Bedeutungen haben, hat, dadurch gekennzeichnet, daß Diazaspirodecane der Formel (VIIIa) oder (VIIIb)

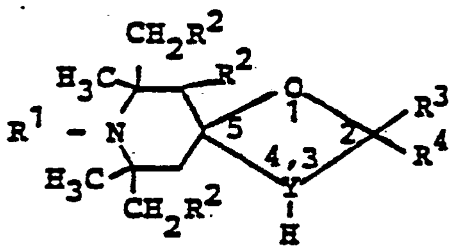

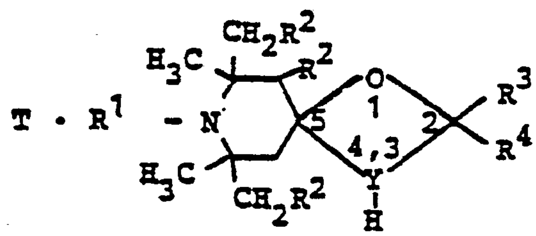

(VIIIa) (VIIIb)

worin $R^1$, $R^2$, $R^3$, $R^4$ und Y die oben angegebenen Bedeutungen haben und T eine nicht oxidierende Mineralsäure oder eine aliphatische oder aromatische Sulfon-oder Phosphonsäure, eine aliphatische Mono-, Di- oder Polycarbonsäure oder eine aromatische Mono-oder Dicarbonsäure ist, und Epoxiden der Formel-(IXa) oder (IXb)

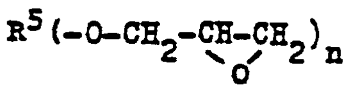

(IXa)

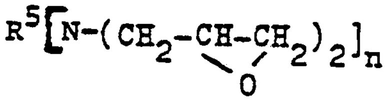

(IXb)

worin $R^5$ und n die oben angegebenen Bedeutungen haben, umgesetzt werden, wobei die Verbindungen-(VIIIa) und (VIIIb) in einem inerten organischen Lösungsmittel in Gegenwart der 0,2-bis 1,5fach äquivalenten Menge einer Base, bezogen auf die Verbindungen (VIIIa), oder der 1,2-bis 2,5fach äquivalenten Menge einer Base bei Einsatz der Verbindung (VIIIb), bei einer Temperatur von 40 bis 180°C mit einem Unterschuß

von einem bis zu einem fünftel Äquivalent, bezogen auf die Epoxidgruppen in den Verbindungen-(IXa) oder (IXb), eingesetzt werden, wobei gegebenenfalls ein Phasentransferkatalysator zugesetzt wird und die so hergestellten Zwischenprodukte anschließend in Substanz oder in Lösung, gegebenenfalls in Gegenwart von Katalysatoren, in höhermolekulare Verbindungen überführt werden.

2. Verwendung der Verbindungen nach Anspruch 1 zum Stabilisieren von synthetischen Polymeren, in einer Menge von 0,01 bis 5 Gew.%, bez auf das zu stabilisierende Material.

3. Verwendung nach Anspruch 2 dadurch gekennzeichnet, daß das Polymere ein Polyolefin, ein Polyacrylat, ein Polymethacrylat, oder ein Homo-oder Copolymerisat des Styrols ist.

4. Verwendung nach Anspruch 2, dadurch gekennzeichnet, daß das Polymere den Feststoffanteil eines Lackes darstellt.

5. Verfahren zum Stabiliseren von synthetischen Polymeren gegen den schädigenden Einfluß von Licht, darduch gekennzeichnet, daß man den Polymeren, gegebenenfalls neben bisher bekannten, stabilisierend wirkenden Stoffen 0,01 bis 5 Gew-% bezogen auf Polymeres, eines Stabilisators nach Anspruch 1 zusetzt.

6. Gegen UV-Zersetzung stabilisierte, synthetische Polymere, in denen 0,01 bis 5 Gew-%, bezogen auf Polymeres, eines Stabilisators nach Anspruch 1 enthalten sind.

## Claims

### Claims for the contracting states BE, CH, DE, FR, GB, IT, LI, NL, SE

1. Compounds of the formula (I)

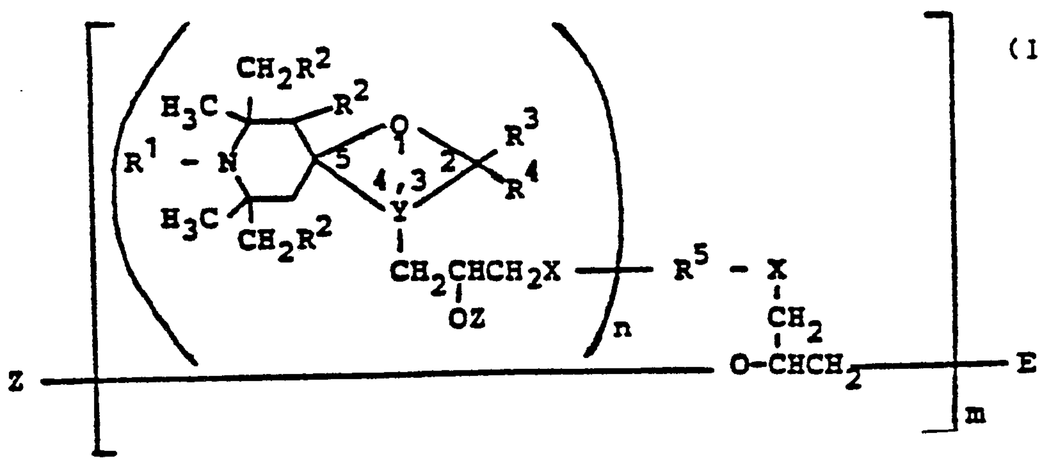

(I)

in which

n is a whole number from 1 to 4,

m is a whole number from 2 to 100,

$R^1$ is hydrogen, $C_1$- to $C_4$-alkyl, benzyl, allyl, $C_2$-to $C_{30}$-alkanoyl, $C_2$- to $C_{20}$-alkenoyl, $C_7$- to $C_{11}$-aroyl, $C_8$- to $C_{14}$-arylalkanoyl or $C_8$- to $C_{20}$-alkylaryl,

$R^2$ is hydrogen or $C_1$- to $C_4$-alkyl,

$R^3$ and $R^4$ are identical or different and denote hydrogen, $C_1$- to $C_{13}$-alkyl, unsubstituted or chlorine- or $C_1$-to $C_4$-alkyl- substituted phenyl, unsubstituted or $C_1$- to $C_4$-alkyl-substituted $C_7$-to $C_{14}$-aralkyl, or together with the carbon atom connecting them denote an unsubstituted or $C_1$-to $C_4$-alkyl-mono-, -di-, -tri- or -tetrasubstituted $C_5$- to $C_{12}$-cycloalkyl or piperidine ring,

$R^5$ when n = 1 denotes $C_2$- to $C_{18}$-alkylene, unsubstituted or $C_1$- to $C_4$-alkyl-mono- or -di substituted phenylene, α, ω-dicarboxy-$C_1$- to $C_8$-alkylene, a dicarboxy-$C_6$-ring, $C_7$- to $C_{14}$-aralkylene, cycloalkylene, dicycloalkylene, tricycloalkylene, bismethylenemonocycloalkylene, bismethylenedicycloalkylene, bismethylenetricycloalkylene, arylene, bisarylenealkyl, which radicals can also be bromine- or chlorine- or C1-C4-alkyl-substituted, a radical

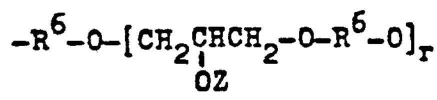

with

r = 1 to 20 and $R^6$ equal to $C_2$- to $C_{12}$-alkylene, cycloalkylene, dicycloalkylene, tricycloalkylene, bismethylenemonocycloalkylene, bismethylenedicycloalkylene, bismethylenetricycloalkylene, arylene, bisaryleneалkyl, which radicals can also be bromineor chlorine- or $C_1$ - to $C_4$-alkyl-substituted,

$R^5$ when n = 2 denotes the radical of a trifunctional alcohol or amine, the trifunctional radical of an aliphatic alcohol which contains further hydroxyl groups, or the trifunctional radical of a novolak based on phenyl, cresol, bisphenol-F or bisphenol-A,

$R^5$ when n = 3 denotes the tetrafunctional radical of an aliphatic alcohol or amine or of a novolak based on phenol, cresol, bisphenol-F or bisphenol-A or the radical

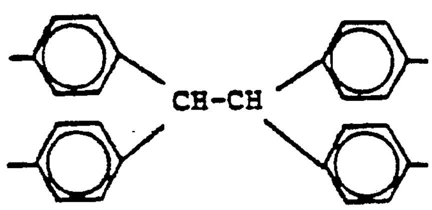

$R^5$ when n = 4 denotes a pentafunctional radical of a polyol, or a polyamine or of a novolak based on phenol, cresol, bisphenol-F or bisphenol-A,

Y denotes

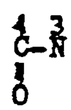 or 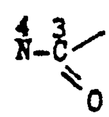

and occupies ring positions 3, 4 in the formula (I),

X represents

-O- or 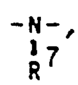

where $R^7$ is hydrogen, $C_1$- to $C_{30}$-alkyl, a piperidine ring which is substituted by 1 to 4 $C_1$- to $C_4$-alkyl groups, or a radical of the formula (II)

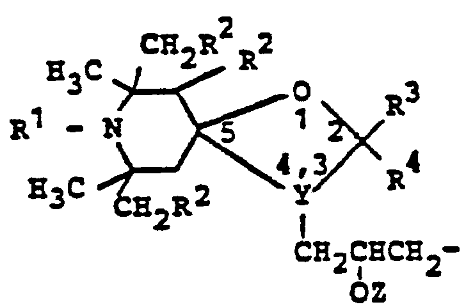

(II)

in which $R^1$, $R^2$, $R^3$, $R^4$, Y have the abovementioned meanings,

Z is hydrogen, a radical of the formula (III)

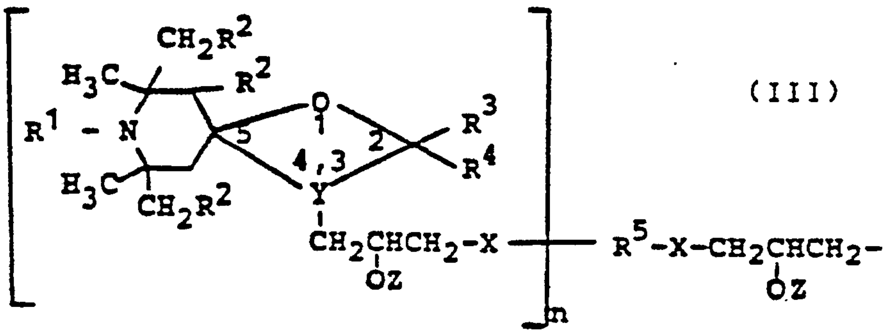

(III)

in which $R^1$, $R^2$, $R^3$, $R^4$, $R^5$, X, Y, Z and n have the abovementioned meanings, or a radical of the formula (II),

E stands for hydrogen, hydroxyl, a radical of the formula (IV), (V) or (VI)

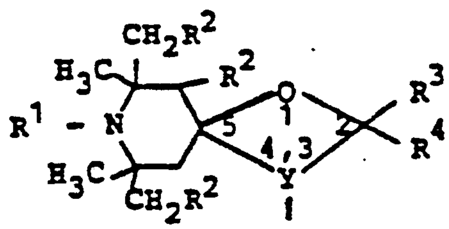

(IV)

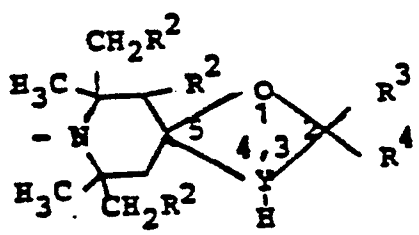

(V)

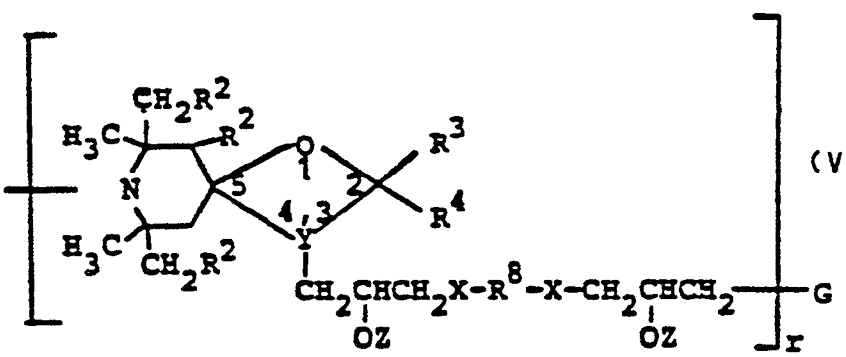

(VI)

in which $R^1$, $R^2$, $R^3$, $R^4$, $R^5$, X, Y and Z have the abovementioned meanings, r represents a whole number from 1 to 100, and G is a radical of the formula (IV) or (V) and $R^8$ has the meaning of formula (VII)

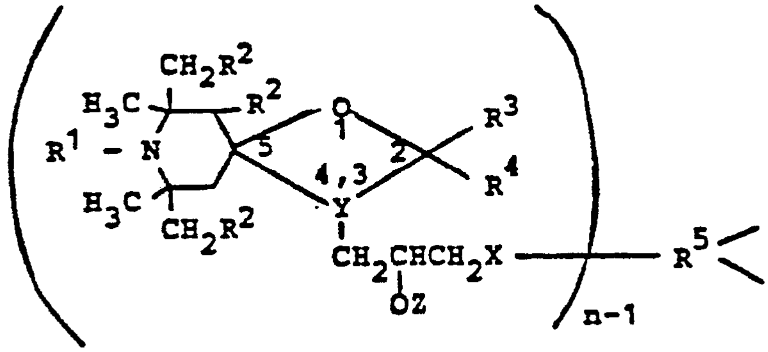

(VII)

in which $R^1$, $R^2$, $R^3$, $R^4$, $R^5$, X, Y, Z and n have the abovementioned meanings.

2. A process for preparing compounds of the formula (I), which comprises reacting diazaspirodecanes of the formula (VIIIa) or (VIIIb)

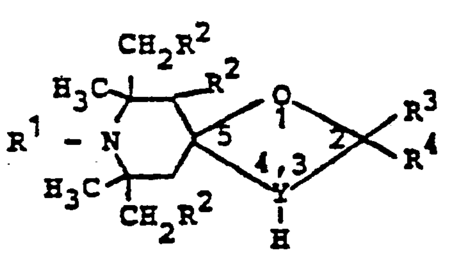

(VIIIa)

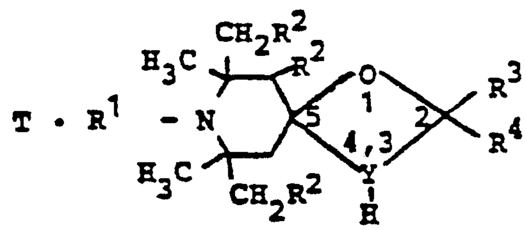

(VIIIb)

in which $R^1$, $R^2$, $R^3$, $R^4$ and Y have the abovementioned meanings and T is a nonoxidizing mineral acid or an aliphatic or aromatic sulfonic or phosphonic acid, an aliphatic mono-, di- or polycarboxylic acid or an aromatic mono- or dicarboxylic acid, and epoxides of the formula (IXa) or (IXb)

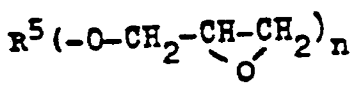

(IXa)

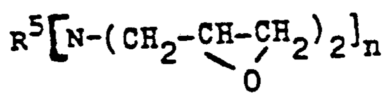

(IXb)

in which $R^5$ and n have the abovementioned meanings, the compounds (VIIIa) and (VIIIb) being used in an inert organic solvent in the presence of 0.2 to 1.5 times the equivalent amount of a base, relative to the compound (VIIIa), or 1.2 to 2.5 times the equivalent amount of a base in the case of using compound (VIIIb), at a temperature of 40 to 180°C with a deficiency of one to a fifth equivalent, based on the epoxy groups in the compounds (IXa) or (IXb), if desired a phase transfer catalyst being added and the intermediates thus prepared being subsequently converted in the presence or absence of a solvent, if desired in the presence of catalysts, into higher molecular weight compounds.

3. Use of the compounds as claimed in claim 1, for stabilizing synthetic polymers in an amount of 0.01 to 5% by weight relative to the material to be stabilized.

4. Use according to claim 3, wherein the polymer is a polyolefin, a polyacrylate, a polymethacrylate or a homopolymer or copolymer of styrene.

5. Use according to claim 3, wherein the polymer is the solids content of a lacquer.

6. A process for stabilizing synthetic polymers against the damaging influence of light, which comprises adding to the polymers, if desired in addition to hitherto disclosed stabilizing substances, 0.01 to 5% by

weight, based on polymer, of the stabilizer as claimed in claim 1.

7. A synthetic polymer stabilized against UV decomposition and containing 0.01 to 5% by weight, based on polymer, of a stabilizer as claimed in claim 1.

**Claims for the contracting state AT**

1. Process for the manufacture of compounds of the formula (I)

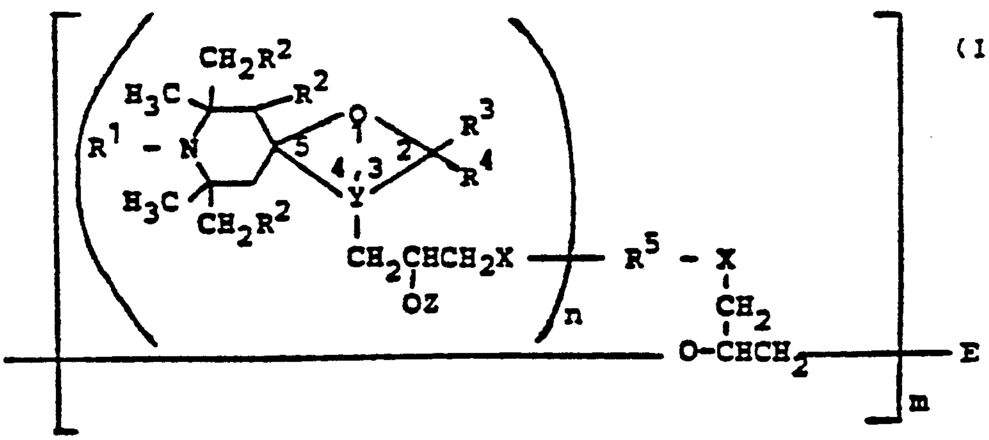

(I)

in which

n is a whole number from 1 to 4,

m is a whole number from 2 to 100,

$R^1$ is hydrogen, $C_1$- to $C_4$-alkyl, benzyl, allyl, $C_2$-to $C_{30}$-alkanoyl, $C_2$- to $C_{20}$-alkenoyl, $C_7$- to $C_{11}$-aroyl, $C_8$- to $C_{14}$-arylalkanoyl or $C_8$- to $C_{20}$-alkylaryl,

$R^2$ is hydrogen or $C_1$- to $C_4$-alkyl,

$R^3$ and $R^4$ are identical or different and denote hydrogen, $C_1$- to $C_{13}$-alkyl, unsubstituted or chlorine- or $C_1$-to $C_4$-alkyl- substituted phenyl, unsubstituted or $C_1$- to $C_4$-alkyl-substituted $C_7$-to $C_{14}$-aralkyl, or together with the carbon atom connecting them denote an unsubstituted or $C_1$-to $C_4$-alkyl-nono-, -di-, -tri- or -tetrasubstituted $C_5$- to $C_{12}$ cycloalkyl or piperidine ring,

$R^5$ when n = 1 denotes $C_2$- to $C_{18}$-alkylene, unsubstituted or $C_1$- to $C_4$-alkyl-mono- or -di substituted phenylene, α, ω-dicarboxy-$C_1$- to $C_8$-alkylene, a dicarboxy-$C_6$-ring, $C_7$- to $C_{14}$-aralkylene, cycloalkylene, dicycloalkylene, tricycloalkylene, bismethylenemonocycloalkylene, bismethylenedicycloalkylene, bismethylenetricycloalkylene, arylene, bisaryleneaIkyl, which radicals can also be bromine- or chlorine- or $C_1$-$C_4$-alkyl-substituted, a radical

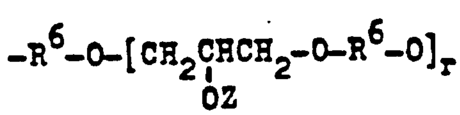

with

r = 1 to 20 and $R^6$ equal to $C_2$- to $C_{12}$-alkylene, cycloalkylene, dicycloalkylene, tricycloalkylene, bismethylenemonocycloalkylene, bismethylenedicycloalkylene, bismethylenetricycloalkylene, arylene, bisaryleneaIkyl, which radicals can also be bromine-or chlorine- or $C_1$- to $C_4$-alkyl-substituted,

$R^5$ when n = 2 denotes the radical of a trifunctional alcohol or amine, the trifunctional radical of an aliphatic alcohol which contains further hydroxyl groups, or the trifunctional radical of a novolak based on phenyl, cresol, bisphenol-F or bisphenol-A,

$R^5$ when n = 3 denotes the tetrafunctional radical of an aliphatic alcohol or amine or of a novolak based on phenol, cresol, bisphenol-F or bisphenol-A or the radical

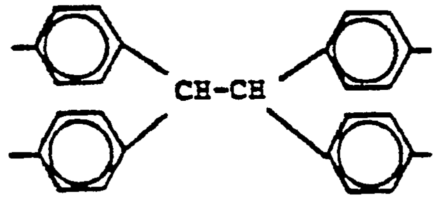

$R^5$ when n = 4 denotes a pentafunctional radical of a polyol, or a polyamine or of a novolak based on phenol, cresol, bisphenol-F or bisphenol-A,

Y denotes

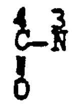 or 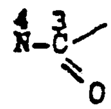

and occupies ring positions 3, 4 in the formula (I),

X represents

-O- or 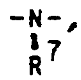

where $R^7$ is hydrogen, $C_1$- to $C_{30}$-alkyl, a piperidine ring which is substituted by 1 to 4 $C_1$- to $C_4$-alkyl groups, or a radical of the formula (II)

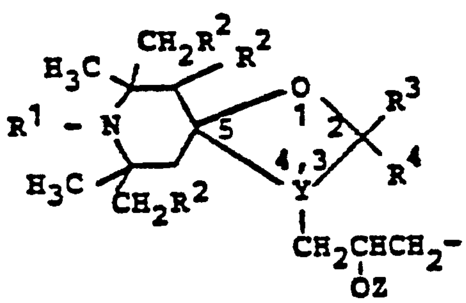

(II)

in which $R^1$, $R^2$, $R^3$, $R^4$, Y have the abovementioned meanings,

Z is hydrogen, a radical of the formula (III)

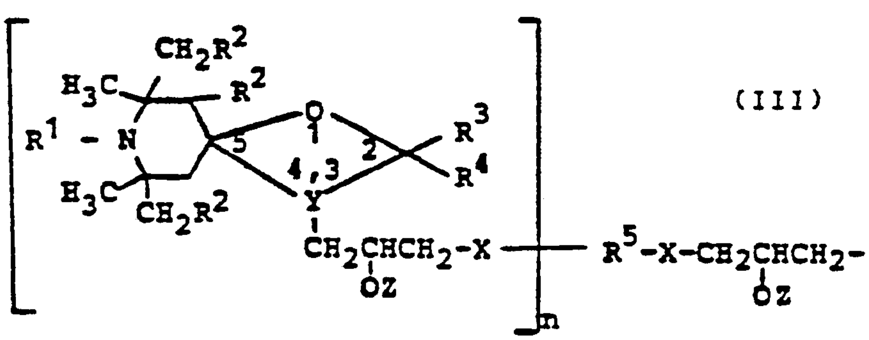

(III)

in which $R^1$, $R^2$, $R^3$, $R^4$, $R^5$, X, Y, Z and n have the abovementioned meanings, or a radical of the formula (II),

E stands for hydrogen, hydroxyl, a radical of the formula (IV), (V) or (VI)

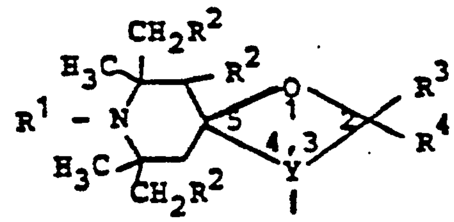

(IV)

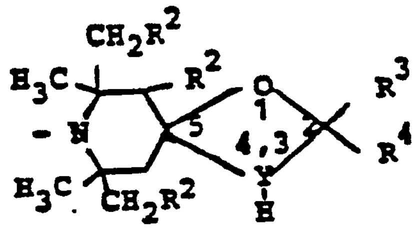

(V)

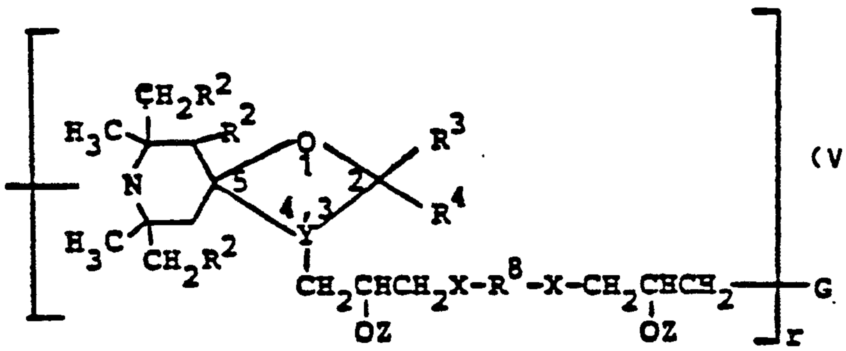

(VI)

in which $R^1$, $R^2$, $R^3$, $R^4$, $R^5$, X, Y and Z have the abovementioned meanings, r represents a whole number from 1 to 100, and G is a radical of the formula (IV) or (V) and R8 has the meaning of formula (VII)

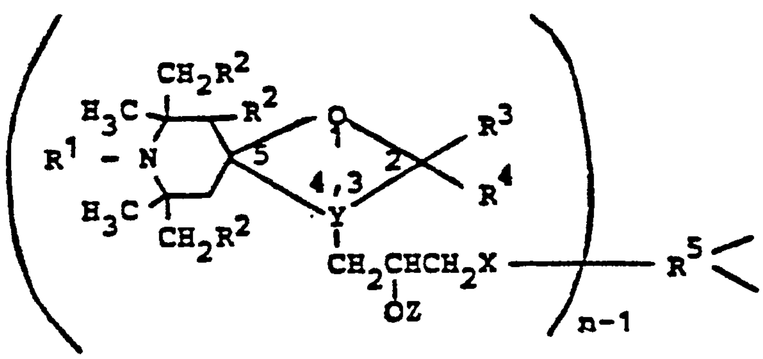

(VII)

in which $R^1$, $R^2$, $R^3$, $R^4$, $R^5$, X, Y, Z and n have the abovementioned meanings, which comprises reacting diazaspirodecanes of the formula (VIIIa) or (VIIIb)

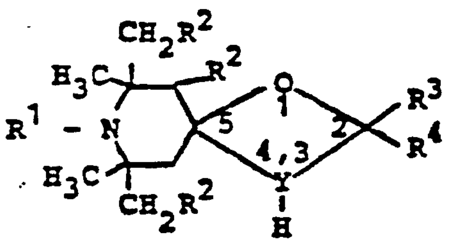

(VIIIa)

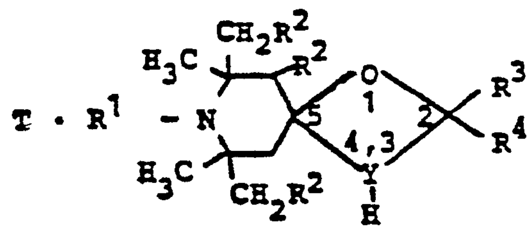

(VIIIb)

in which $R^1$, $R^2$, $R^3$, $R^4$ and Y have the abovementioned meanings and T is a nonoxidizing mineral acid or an aliphatic or aromatic sulfonic or phosphonic acid, an aliphatic mono-, di- or polycarboxylic acid or an aromatic mono- or dicarboxylic acid, and epoxides of the formula (IXa) or (IXb)

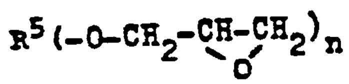

(IXa)

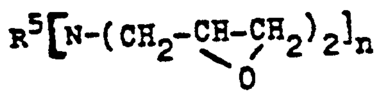

(IXb)

in which $R^5$ and n have the abovementioned meanings, the compounds (VIIIa) and (VIIIb) being used in an inert organic solvent in the presence of 0.2 to 1.5 times the equivalent amount of a base, relative to the compound (VIIIa), or 1.2 to 2.5 times the equivalent amount of a base in the case of using compound (VIIIb), at a temperature of 40 to 180°C with a deficiency of one to a fifth equivalent, based on the epoxy groups in the compounds (IXa) or (IXb), if desired a phase transfer catalyst being added and the intermediates thus prepared being subsequently converted in the presence or absence of a solvent, if desired in the presence of catalysts, into higher molecular weight compounds.

2. Use of the compounds as claimed in claim 1, for stabilizing synthetic polymers in an amount of 0.01 to 5% by weight relative to the material to be stabilized.

3. Use according to claim 2, wherein the polymer is a polyolefin, a polyacrylate, a polymethacrylate or a homopolymer or copolymer of styrene.

4. Use according to claim 2, wherein the polymer is the solids content of a lacquer.

5. A process for stabilizing synthetic polymers against the damaging influence of light, which comprises adding to the polymers, if desired in addition to hitherto disclosed stabilizing substances, 0.01 to 5% by weight, based on polymer, of the stabilizer as claimed in claim 1.

6. A synthetic polymer stabilized against UV decomposition and containing 0.01 to 5% by weight, based on polymer, of a stabilizer as claimed in claim 1.

## Revendications

### Revendications pour les Etats contractants : BE, CH, DE, FR, GB, IT, LI, NL, SE

1. Composés répondant à la formule I :

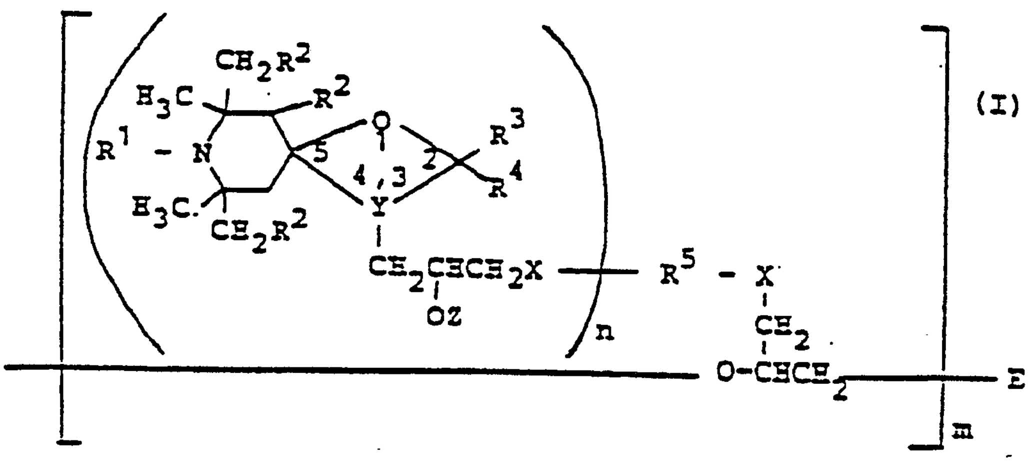

(I)

dans laquelle

n désigne un nombre entier de 1 à 4,

m désigne un nombre entier de 2 à 100,

$R^1$ représente l'hydrogène, un alkyle en $C_1$-$C_4$, un benzyle, un allyle, un alcanoyle en $C_2$-$C_{30}$, un alcénoyle en $C_2$-$C_{20}$, un aroyle en $C_7$-$C_{11}$, un arylalcanoyle en $C_8$-$C_{14}$ ou un alkylaryle en $C_8$-$C_{20}$,

$R^2$ représente l'hydrogène ou un alkyle en $C_1$-$C_4$,

$R^3$ et $R^4$ représentent chacun, indépendamment l'un de l'autre, l'hydrogène, un alkyle en $C_1$-$C_{13}$, un phényle non substitué ou porteur d'un atome de chlore ou d'un alkyle en $C_1$-$C_4$, un aralkyle en $C_7$-$C_{14}$ non substitué ou porteur d'un alkyle en $C_1$-$C_4$, ou forment ensemble, et avec l'atome de carbone qui les unit, un cycle de cycloalcane en $C_5$-$C_{12}$ ou de pipéridine non substitué ou porteur d'au plus 4 alkyles en $C_1$-$C_4$,

$R^5$ représente :

– dans le cas où n est égal à 1, un alkylène en $C_2$-$C_{18}$, un phénylène non substitué ou porteur d'au plus deux alkyles en $C_1$-$C_4$, un α, ω-dicarboxy-alkylène dont la partie alkylène contient de 1 à 8 atomes de carbone, un radical cyclique en $C_6$ porteur de deux radicaux carboxy, un aralkylène en $C_7$-$C_{14}$, un cycloalkylène, un dicycloalkylène, un tricycloalkylène, un bis-méthylène-monocycloalkylène, un bis-méthylènedicycloalkylène, un bis-méthylène-tricycloalkylène, un arylène, un bis-arylène-alkyle - ces radicaux pouvant également porter un atome de brome, un atome de chlore ou des alkyles en $C_1$-$C_4$ -, un radical

$$-R^6-O-[CH_2-\underset{\displaystyle OZ}{\underset{|}{C}}HCH_2-O-R^6-O]_r$$

(dans lequel r désigne un nombre de 1 à 20 et $R^6$ représente un alkylène en $C_2$-$C_{12}$, un cycloalkylène, un dicycloalkylène, un tricycloalkylène, un bis-méthylènemonocycloalkylène, un bis-méthylène-dicycloalkylène, un bisméthylène-tricycloalkylène, un arylène ou un bis-arylènealkyle, ces radicaux pouvant également porter un atome de brome, un atome de chlore ou des alkyles en $C_1$-$C_4$),

– dans le cas où n est égal à 2, le radical d'un alcool trifonctionnel ou d'une amine trifonctionnelle, le radical trifonctionnel d'un alcool aliphatique contenant encore des radicaux hydroxy, ou le radical trifonctionnel d'une novolaque dérivant du phénol, du crésol, du bis-phénol F ou du bis-phénol A,

– dans le cas où n est égal à 3, le radical tétrafonctionnel d'un alcool aliphatique ou d'une amine aliphatique, ou d'une novolaque dérivant du phénol, du crésol, du bis-phénol F ou du bis-phénol A, ou le radical :

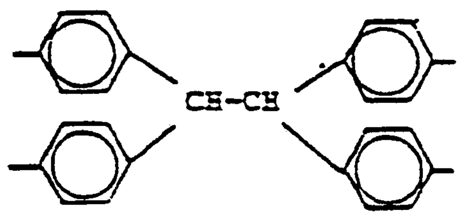

– dans le cas où n est égal à 4, un radical pentafonctionnel d'un polyol, d'une polyamine ou d'une novolaque dérivant du phénol, du crésol, du bis-phénol F ou du bis-phénol A,

Y représente un radical

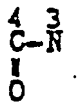 ou 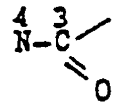

occupant les positions 3 et 4 du cycle dans la formule I,

X représente

-O- ou 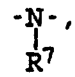,

le symbole $R^7$ représentant l'hydrogène, un alkyle en $C_1$-$C_{30}$, un cycle pipéridinique porteur d'un à quatre alkyles en $C_1$-$C_4$, ou un radical de formule II :

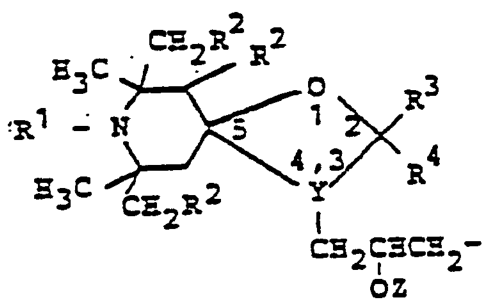

(II)

dans lequel $R^1$, $R^2$, $R^3$, $R^4$ et Y ont les significations indiquées ci-dessus,

Z représente l'hydrogène, un radical de formule III

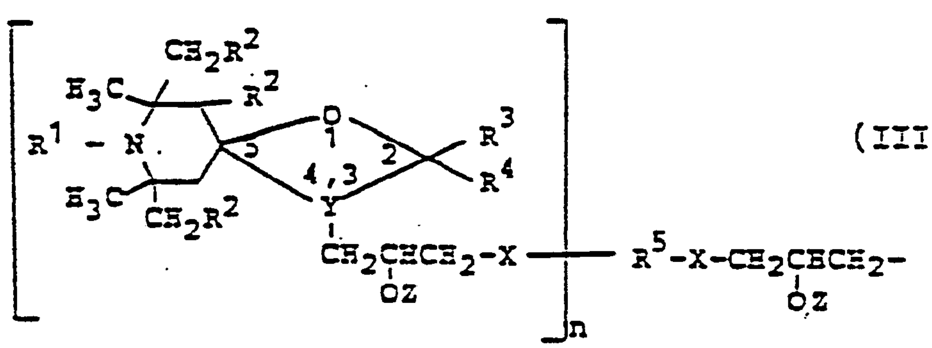

(III)

dans lequel $R^1$, $R^2$, $R^3$, $R^4$, $R^5$, X, Y, Z et n ont les significations indiquées ci-dessus, ou un radical de formule II,

et

E représente l'hydrogène, un hydroxy, ou un radical répondant à l'une des formules IV, V et VI :

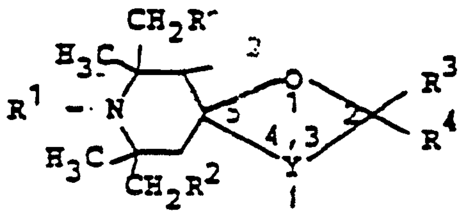

(IV)

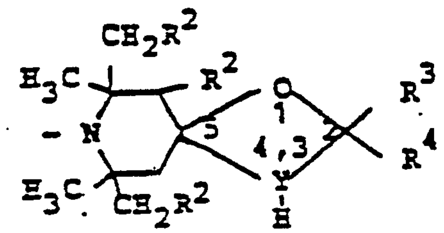

(V)

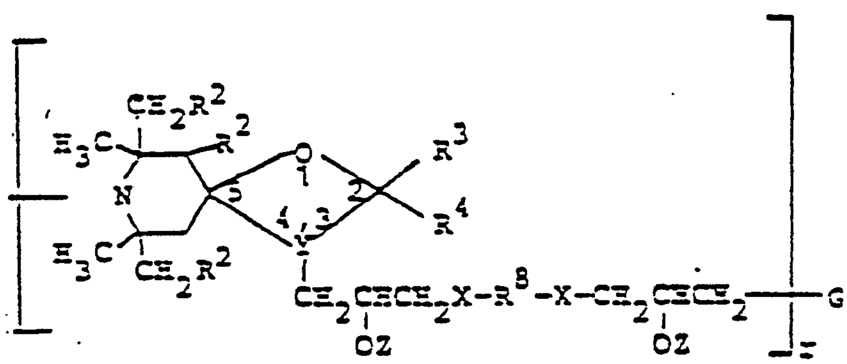

(VI)

dans lesquelles $R^1$, $R^2$, $R^3$, $R^4$, $R^5$, X, Y et Z ont les significations indiquées ci-dessus, r désigne un nombre entier de 1 à 100, G représente un radical de formule IV ou V, et $R^8$ représente un radical de formule VII :

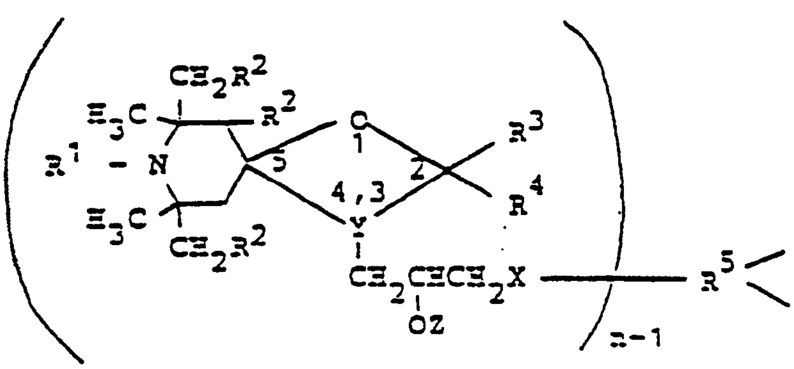

(VII)

dans lequel $R^1$, $R^2$, $R^3$, $R^4$, $R^5$, X, Y, Z et n ont les significations indiquées ci-dessus.

2. Procédé pour préparer des composés de formule générale I, procédé caractérisé en ce qu'on fait

réagir des diaza-oxaspirodécanes répondant à l'une des formules VIIIa et VIIIb :

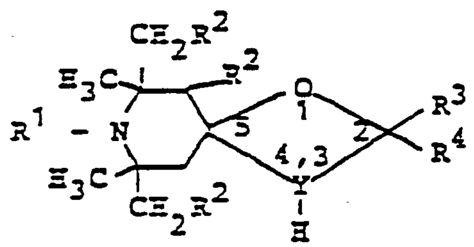

T

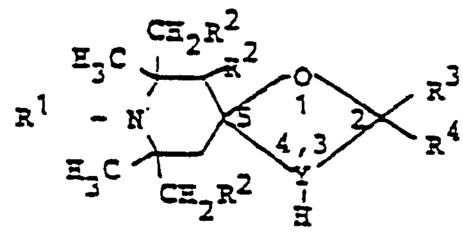

(VIIIa) (VIIIb)

dans lesquelles $R^1$, $R^2$, $R^3$, $R^4$ et Y ont les significations indiquées ci-dessus et T représente un acide minéral non oxydant ou un acide sulfonique ou phosphonique aliphatique ou aromatique, un acide mono-, di- ou polycarboxylique alphatique ou un acide mono- ou dicarboxylique aromatique, et des époxydes répondant à l'une des formules IXa et IXb :

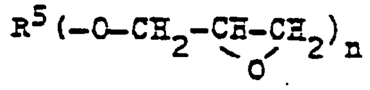

(IXa)

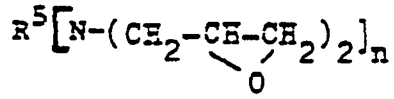

(IXb)

dans lesquelles $R^5$ et n ont les significations indiquées ci-dessus, réaction pour laquelle on utilise les composés (VIIIa) et (VIIIb) dans un solvant organique inerte, en présence de 0,2 à 1,5 équivalent d'une base, par rapport au composé VIIIa, ou de 1,2 à 2,5 équivalents d'une base lorsqu'on se sert du composé (VIIIb), à une température de 40 à 180°C, avec un déficit d'un à un cinquième d'équivalent, par rapport aux radicaux époxy contenus dans les composés (IXa) ou (IXb), et on ajoute éventuellement un catalyseur de transfert de phase, après quoi on transforme les corps intermédiaires ainsi préparés, en substance ou en solution, éventuellement en présence de catalyseurs, en des composés à poids moléculaire plus élevé.

3. Application des composés selon la revendication 1 à la stabilisation de polymères synthétiques, en une quantité de 0,01 à 5% en poids par rapport à la matière à stabiliser.

4. Application selon la revendication 3, caractérisée en ce que le polymère est une polyoléfine, un polyacrylate, un polyméthacrylate ou un homopolymère ou copolymère du styrène.

5. Application selon la revendication 3, caractérisée en ce que le polymère est la partie solide d'un vernis.

6. Procédé pour stabiliser des polymères synthétiques contre l'influence destructrice de la lumière, procédé caractérisé en ce qu'on ajoute aux polymères, éventuellement en plus de composés à action stabilisante connus jusqu'à présent, de 0,01 à 5% en poids, par rapport au polymère, d'un stabilisant selon la revendication 1.

7. Polymères synthétiques stabilisés contre la décomposition par les ultraviolets, polymères synthétiques qui contiennent de 0,01 à 5% en poids, par rapport au polymère, d'un stabilisant selon la revendication 1.

**Revendications pour l'état contractant AT**

1. Procédé pour préparer des composés répondant à la formule I :

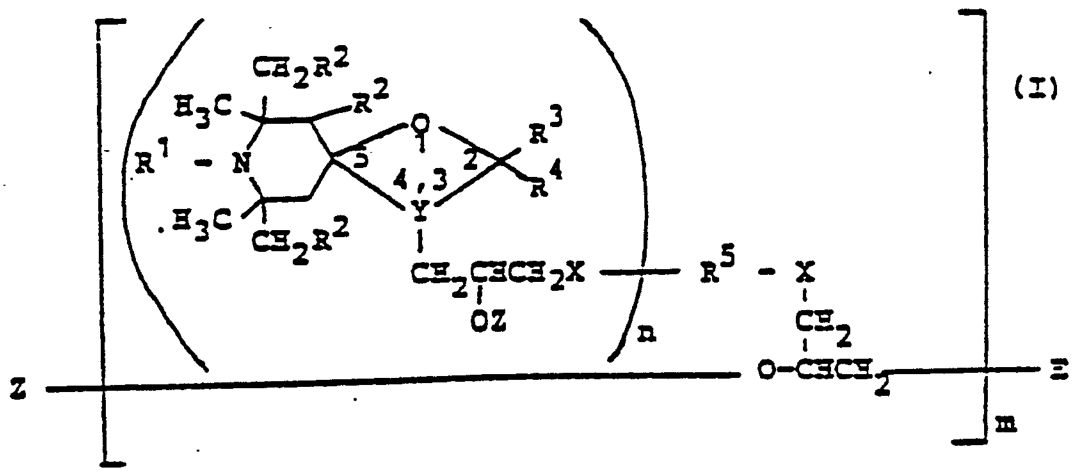

(I)

dans laquelle

n désigne un nombre entier de 1 à 4,

m désigne un nombre entier de 2 à 100

$R^1$ représente l'hydrogène, un alkyle en $C_1$-$C_4$, un benzyle, un allyle, un alcanoyle en $C_2$-$C_{30}$, un alcénoyle en $C_2$-$C_{20}$, un aroyle en $C_7$-$C_{11}$, un arylalcanoyle en $C_8$-$C_{14}$ ou un alkylaryle en $C_8$-$C_{20}$,

$R^2$ représente l'hydrogène ou un alkyle en $C_1$-$C_4$,

$R^3$ et $R^4$ représentent chacun, indépendamment l'un de l'autre, l'hydrogène, un alkyle en $C_1$-$C_{13}$, un phényle non substitué ou porteur d'un atome de chlore ou d'un alkyle en $C_1$-$C_4$, un aralkyle en $C_7$-$C_{14}$ non substitué ou porteur d'un alkyle en $C_1$-$C_4$, ou forment ensemble, et avec l'atome de carbone qui les unit, un cycle de cycloalcane en $C_6$-$C_{12}$ ou de pipéridine non substitué ou porteur d'au plus quatre alkyles en $C_1$-$C_4$,

$R^5$ représente :

– dans le cas où n est égal à 1, un alkylène en $C_2$-$C_{18}$, un phénylène non substitué ou porteur d'au plus deux alkyles en $C_1$-$C_4$, un α, ω-dicarboxy-alkylène dont la partie alkylène contient de 1 à 8 atomes de carbone, un radical cyclique en $C_6$ porteur de deux radicaux carboxy, un aralkylène en $C_7$-$C_{14}$, un cycloalkylène, un dicycloalkylène, un tricycloalkylène, un bis-méthylène-monocycloalkylène, un bis-méthylènedicycloalkylène, un bis-méthylène-tricycloalkylène, un arylène, un bis-arylène-alkyle - ces radicaux pouvant également porter un atome de brome, un atome de chlore ou des alkyles en $C_1$-$C_4$ -, un radical

$$-R^6-O-[CH_2-\underset{OZ}{\underset{|}{C}}HCH_2-O-R^6-O]_r$$

(dans lequel r désigne un nombre de 1 à 20 et $R^6$ représente un alkylène en $C_2$-$C_{12}$, un cycloalkylène, un dicycloalkylène, un tricycloalkylène, un bis-méthylène-monocycloalkylène, un bis-méthylène-dicycloalkylène, un bis-méthylène-tricycloalkylène, un arylène ou un bis-arylène-alkyle, ces radicaux pouvant également porter un atome de brome, un atome de chlore ou des alkyles en $C_1$-$C_4$),

– dans le cas où n est égal à 2, le radical d'un alcool trifonctionnel ou d'une amine trifonctionnelle, le radical trifonctionnel d'un alcool aliphatique contenant encore des radicaux hydroxy, ou le radical trifonctionnel d'une novolaque dérivant du phénol, du crésol, du bis-phénol F ou du bis-phénol A,

– dans le cas où n est égal à 3, le radical tétrafonctionnnel d'un alcool aliphatique ou d'une amine aliphatique, ou d'une novolaque dérivant du phénol, du crésol, du bis-phénol F ou du bis-phénol A, ou le radical :

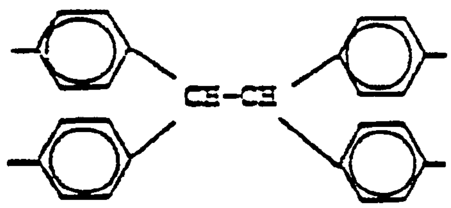

– dans le cas où n est égal à 4, un radical pentafonctionnel d'un polyol, d'une polyamine ou d'une novolaque dérivant du phénol, du crésol, du bis-phénol F ou du bis-phénol A,

Y représente un radical

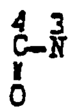 ou 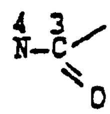

occupant les positions 3 et 4 du cycle dans la formule I,

X représente

-O- ou 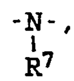,

le symbole $R^7$ représentant l'hydrogène, un alkyle en $C_1$-$C_{30}$, un cycle pipéridinique porteur d'un à quatre alkyles en C1-C4, ou un radical de formule II :

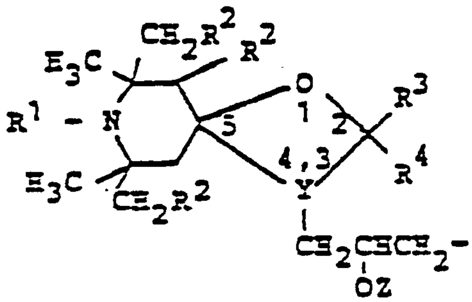 (II)

dans lequel $R^1$, $R^2$, $R^3$, $R^4$ et Y ont les significations indiquées ci-dessus,

Z représente l'hydrogène, un radical de formule III

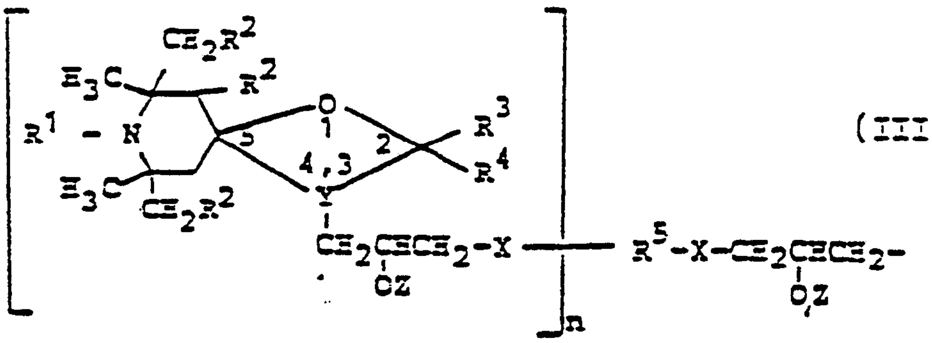

(III)

dans lequel $R^1$, $R^2$, $R^3$, $R^4$, $R^5$, X, Y, Z et n ont les significations indiquées ci-dessus ; ou un radical de formule II,

et

E représente l'hydrogène, un hydroxy, ou un radical répondant à l'une des formules IV, V et VI

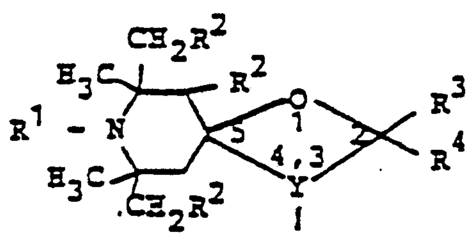

(IV)

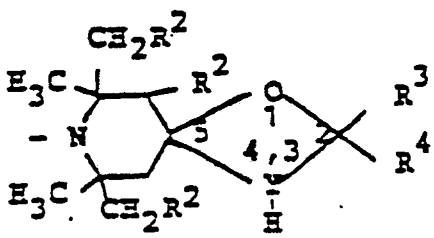

(V)

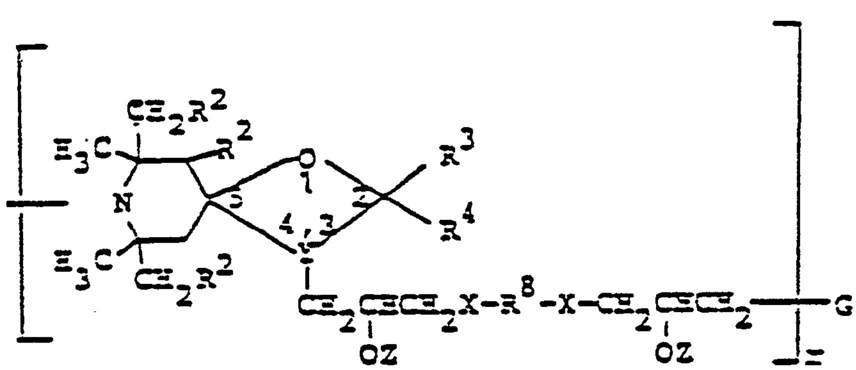

(VI)

dans lesquelles $R^1$, $R^2$, $R^3$, $R^4$, $R^5$, X, Y et Z ont les significations indiquées ci-dessus, r désigne un nombre entier de 1 à 100, G représente un radical de formule IV ou V, et $R^8$ représente un radical de formule VII :

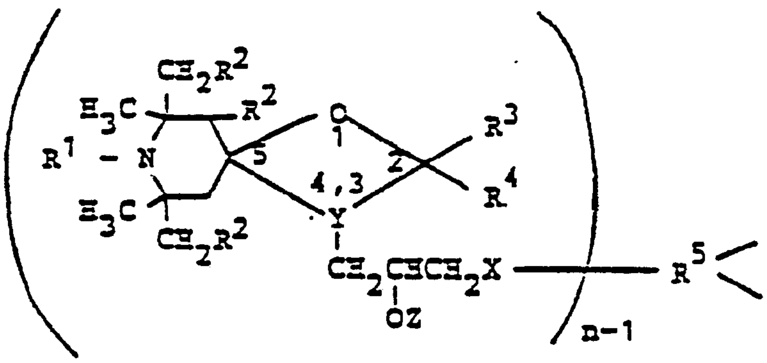

(VII)

dans lequel $R^1$, $R^2$, $R^3$, $R^4$, $R^5$, X, Y, Z et n ont les significations indiquées ci-dessus, procédé caractérisé en ce qu'on fait réagir des diaza-spirodécanes répondant à l'une des formules VIIIa et VIIIb :

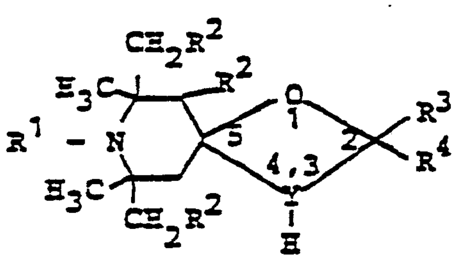

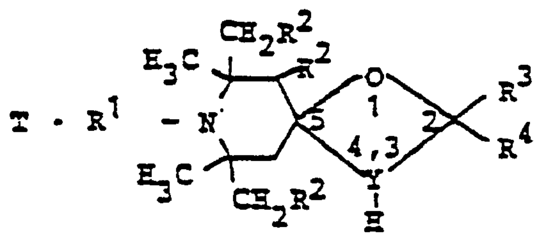

(VIIIa) (VIIIb)

dans lesquelles $R^1$, $R^2$, $R^3$, $R^4$ et Y ont les significations indiquées ci-dessus et T représente un acide minéral non oxydant ou un acide sulfonique ou phosphonique aliphatique ou aromatique, un acide mono-, di- ou polycarboxylique aliphatique ou un acide mono- ou dicarboxylique aromatique, et des époxydes répondant à l'une des formules IXa et IXb :

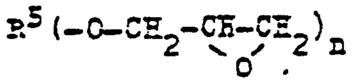

(IXa)

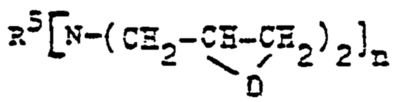

(IXb)

dans lesquelles $R^5$ et n ont les significations indiquées ci-dessus, réaction pour laquelle on utilise les composés (VIIIa) et (VIIIb) dans un solvant organique inerte, en présence de 0,2 à 1,5 équivalent d'une base, par rapport au composé VIIIa, ou de 1,2 à 2,5 équivalents d'une base lorsqu'on se sert du composé (VIIIb), à une température de 40 à 180°C, avec un déficit d'un à un cinquième d'équivalent, par rapport aux radicaux époxy contenus dans les composés (IXa) ou (IXb), et on ajoute éventuellement un catalyseur de transfert de phase, après quoi on transforme les corps intermédiaires ainsi préparés, en substance ou en solution,

éventuellement en présence de catalyseurs, en des composés à poids moléculaire plus élevé.

2. Application des composés selon la revendication 1 à la stabilisation de polymères synthétiques, en une quantité de 0,01 à 5% en poids par rapport à la matière à stabiliser.

3. Application selon la revendication 2, caractérisée en ce que le polymère est une polyoléfine, un polyacrylate, un polyméthacrylate ou un homopolymère ou copolymère du styrène.

4. Application selon la revendication 2, caractérisée en ce que le polymère est la partie solide d'un vernis.

5. procédé pour stabiliser des polymères synthétiques contre l'influence destructrice de la lumière, procédé caractérisé en ce qu'on ajoute aux polymères, éventuellement en plus de composés à action stabilisante connus jusqu'à présent, de 0,01 à 5% en poids, par rapport au polymère, d'un stabilisant selon la revendication 1.

6. Polymères synthétiques stabilisés contre la décomposition par les ultraviolets, polymères synthétiques, qui contiennent de 0,01 à 5% en poids, par rapport au polymère, d'un stabilisant selon la revendication 1.